# EUROPEAN PATENT SPECIFICATION

(11) **EP 4 297 666 B1**
(45) Date of publication and mention of the grant of the patent: **14.10.2026**
(21) Application number: 22718338.1
(22) Date of filing: 17.02.2022
(51) Int. Cl.: A61B 17/16, A61B 17/88

(54) **SURGERY INSTRUMENTS WITH A MOVABLE HANDLE**
CHIRURGISCHE INSTRUMENTE MIT EINEM BEWEGLICHEN GRIFF
INSTRUMENTS CHIRURGICAUX À POIGNÉE MOBILE

(30) Priority: 25.02.2021 US 202117185062
(43) Date of publication of application: 03.01.2024
(73) Proprietor: Warsaw Orthopedic, Inc., Warsaw, IN 46582 (US)
(72) Inventor: NAYET, Jerome, Minneapolis, Minnesota 55432 (US); JOSSE, Loic, Minneapolis, Minnesota 55432 (US); HYNES, Richard A. Hynes, Minneapolis, Minnesota 55432 (US)
(74) Representative: Viering, Jentschura & Partner mbB Patent- und Rechtsanwälte
(86) International application number: PCT/US2022/016831
(87) International publication number: WO 2022/182569

(56) References cited:
- EP-A2- 2 954 860
- WO-A1-2014/145478
- US-A1- 2008 058 804
- US-A1- 2010 076 440
- US-A1- 2017 071 631
- US-A1- 2019 262 009
- US-A1- 2019 329 388

## Description

### FIELD

The present technology is generally related to screwdrivers for installing, removing, and/or manipulating complimentary bone screws.

### BACKGROUND

The installation and insertion of bone screws in a patient poses many risks. At least one risk is the pre-operative step of drilling a passageway for a bone screw safely within a surgical opening of a patient, for example. Conventional drills may have sharp edges and a cutting tip that can cause accidental injuries to a patient. For example, in operation, an edge of a drill bit may catch an adjacent surface or "walk" away from an intended drill site and damage adjacent soft tissues. These problems may occur in all surgical settings requiring a drill although are particularly problematic in mini open surgeries and other minimally invasive surgical techniques, for example.

The installation and insertion of bone screws in a patient poses many risks. Some of these risks may include the loss of a bone screw in the patient, which can have dramatic consequences and even lead to death in some cases. Additionally, conventional screwdrivers and related tools are often unsuitable for avoiding anatomical features, such as the pelvic ring, rib cage, iliac crest, etc., for example. Additionally, conventional screwdrivers are not be well suited for installation of bone screws in angled bone plates and/or angled apertures of spinal implants, for example. There is a need for screwdrivers that can securely connect to a complimentary bone screw with sufficient force that the bone screw will not become accidentally detached during the initial positioning and installation of the bone screw. Additionally, there is a need for screwdrivers that are optimized for the installation of bone screws to secure spinal implants to adjacent vertebrae of a patient according to various surgical techniques including anterior techniques, lateral techniques, and oblique techniques.

The length of surgical instruments is usually dictated by the approach and methodology. For example, a posterior approach versus anterior approach and a lumbar approach versus a cervical approach, for example. Conventional surgical instruments are relatively long and may not be optimal with all patient anatomy and sizes and/or operator anatomy and sizes. At least one drawback of certain conventional surgical tools is that handles are fixed and typically positioned far away from the distal end thus reducing the precision of use which can lead to taking a longer time for certain surgeries and require additional care to avoid risks to the patient.

Further to the above, US 2008/058804 A1 discloses a precision adjustable surgical tool holder/driver, which is easily disassembled for cleaning and precisely reassembled, wherein the holder/driver has a positionable handle allowing adjustment of the position of the handle about the spindle of the holder to enable the handedness of the holder to be changed in order to accommodate a user while operating from the left or right side of the patient, standing behind or in-front of the patient, or for use in different surgical methods, wherein the holder/driver includes a limited-play capture mechanism, which connects the drive end of the housing to its locking sleeve via a limited-play locking device, wherein the limited-play locking device utilizes a disengageable, precision fitted bayonet pin and seat combination in part to accomplish the precision reassembly feature disclosed in US 2008/058804 A1.

Further surgical drills and screwdrivers as well related techniques are known from US 2010/076440 A1, EP 2 954 860 A2 and US 2019/329388 A1.

### SUMMARY

The present invention provides a screwdriver as set out by claim 1. Further advantageous embodiments are described in the dependent claims.

According to the present disclosure a screwdriver is provided, including: a rotatable drive shaft, the drive shaft including a drive portion disposed at a distal end thereof, a drive end disposed at a proximal end thereof, and a main shaft portion extending in a longitudinal direction through a housing; a movable handle mechanism coupled to and disposed at a medial portion of the housing; an angled tip portion disposed at the proximal end, the angled tip portion being angled with respect to the longitudinal direction, the drive end of the drive shaft extending through the angled tip portion; and a mechanism configured to transfer a rotational force applied to the drive portion of the drive shaft through the angled tip portion to the drive end of the drive shaft. The screwdriver further includes: an elastic retaining clip configured to have a bone screw securely attached therein at a clipping force and progressively release the bone screw therein at an extraction force, the elastic retaining clip being removably and operably coupled with the drive end of the drive shaft; and a first spring contacting the elastic retaining clip and the angled tip portion, the first spring being configured to facilitate the progressive release of the bone screw.

In another aspect, the disclosure provides that the movable handle mechanism includes a positioning handle, for example.

In another aspect, the disclosure provides that the movable handle mechanism may be configured to move forward and backward in a longitudinal direction along the housing.

In another aspect, the disclosure provides that the housing includes a plurality of channels and the movable handle mechanism is configured to securely couple to the housing via at least one channel of the plurality of channels, for example.

In another aspect, the disclosure provides that the movable handle mechanism is configured to rotate clockwise and counterclockwise around the housing, for example.

In another aspect, the disclosure provides that the housing includes a plurality of detents, and the movable handle mechanism is configured to securely couple to the housing via at least one detent of the plurality of detents, for example.

In another aspect, the disclosure provides that the movable handle mechanism further comprises a positioning ball having a size and shape corresponding to a size and shape of a detent of the plurality of detents, for example. In various embodiments, the positioning ball may be configured to securely couple the movable handle mechanism via one detent of the plurality of detents, for example.

In another aspect, the disclosure provides that the movable handle mechanism is configured to move forward and backward in a longitudinal direction along the housing between a proximal stop ring and a distal stop ring and rotate clockwise and counterclockwise around the housing, for example.

In another aspect, the disclosure provides that the housing may include a plurality of channels, and each channel of the plurality of channels may include at least one detent, for example. In another aspect, the disclosure provides that the movable handle mechanism may be configured to securely couple to the housing via one channel of the plurality of channels and one detent of the plurality of detents, for example.

In another aspect, the disclosure provides that the movable handle mechanism may include a positioning ball having a size and shape corresponding to a size and shape of a detent of the plurality of detents, for example. In various embodiments, the positioning ball may be configured to securely couple the movable handle mechanism via one detent of the plurality of detents, for example.

In another aspect, the disclosure provides that the movable handle mechanism is configured to move forward and backward in a longitudinal direction along the housing between a proximal stop ring and a distal stop ring, for example. In various embodiments, the movable handle mechanism is configured to rotate clockwise and counterclockwise around the housing, for example. In various embodiments, the housing includes a plurality of channels, each channel of the plurality of channels including at least one detent, and the movable handle mechanism is configured to securely couple to the housing via at least one channel of the plurality of channels and at least one detent of the plurality of detents, for example.

In another aspect, the present disclosure provides that the screwdriver further may include a second spring configured to facilitate the progressive release of the bone screw, that the first spring and second spring each contact the elastic retaining clip and the angled tip portion, and that the first spring is disposed, at least partly, within a central cavity of the second spring.

In another aspect, the present disclosure provides that the at least one protrusion may be radially inset with respect to a head portion of the bone screw and is configured to contact an end portion of the head portion of the bone screw to thereby facilitate the retention of the bone screw.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

In the following, Figs. 1 to 14 may not form part of the claimed invention as such, but are considered as useful for understanding the invention.
FIG. 1 is a perspective view of an example drill in accordance with the principles of the present disclosure;
FIG. 2A is an alternate perspective view of an example drill in accordance with the principles of the present disclosure;
FIG. 2B is a top down view of an example drill in accordance with the principles of the present disclosure;
FIG. 2C is a top down view of a housing of a surgical tool in accordance with the principles of the present disclosure;
FIG. 2D is a perspective view of an example movable handle mechanism in accordance with the principles of the present disclosure;
FIG. 2E is an exploded parts view of an example movable handle mechanism in accordance with the principles of the present disclosure;
FIG. 3 is a magnified view of the tip portion of an example drill in accordance with the principles of the present disclosure;
FIG. 4A is a top down view of a flushing portion of an example drill in accordance with the principles of the present disclosure;
FIG. 4B is a top down view of a flushing portion of an example drill with partially removed parts for ease of understanding in accordance with the principles of the present disclosure;
FIG. 5 is a side view of a gear mechanism in accordance with the principles of the present disclosure;
FIG. 6A is a top view of a gear mechanism in accordance with the principles of the present disclosure;
FIG. 6B is a bottom view of a gear mechanism in accordance with the principles of the present disclosure;
FIG. 7 is a cross sectional view of a gear mechanism in accordance with the principles of the present disclosure;
FIG. 8 is a perspective view of a portion of a gear mechanism and a drill bit in accordance with the principles of the present disclosure;
FIG. 9 is a perspective view of a portion of a gear mechanism and an aperture for receiving a drill bit in accordance with the principles of the present disclosure;
FIG. 10 is a side view of an example drill coupled to a powered driver in accordance with the principles of the present disclosure;
FIG. 11 is a side view of an example drill coupled to a manual hand driver in accordance with the principles of the present disclosure;
FIG. 12 is a side view of an example drill engaged with an aperture of an implant in accordance with the principles of the present disclosure;
FIG. 13A is a perspective view of an example implant having conically shaped apertures for seating a tip of a drill in accordance with the principles of the present disclosure;
FIG. 13B is an alternate perspective view of an example implant having conically shaped apertures for seating a tip of a drill in accordance with the principles of the present disclosure;
FIG. 14 is an example flow chart of a method which does not form part of the invention as claimed;
FIG. 15 is a perspective view of an example screwdriver in accordance with the principles of the present disclosure;
FIG. 16 is a side view of an example screwdriver in accordance with the principles of the present disclosure;
FIG. 17 is a magnified view of the tip portion of an example screwdriver in accordance with the principles of the present disclosure;
FIG. 18A is a top down view of a flushing portion of an example screwdriver in accordance with the principles of the present disclosure;
FIG. 18B is a top down view of a flushing portion of an example screwdriver with partially removed parts for ease of understanding in accordance with the principles of the present disclosure;
FIG. 19 is a perspective view of an example bone screw suitable for use with disclosed embodiments in accordance with the principles of the present disclosure;
FIG. 20 is a perspective view of an example bone screw suitable for use with disclosed embodiments in accordance with the principles of the present disclosure;
FIG. 21 is a perspective view of an example bone screw suitable for use with disclosed embodiments in accordance with the principles of the present disclosure;
FIG. 22 is a perspective view of an example bone screw coupled to a retaining cap in accordance with the principles of the present disclosure;
FIG. 23A is a perspective view of a retaining cap viewed from a drive shaft connecting side in accordance with the principles of the present disclosure;
FIG. 23B is an alternate perspective view of a retaining cap viewed from a drive shaft connecting side in accordance with the principles of the present disclosure;
FIG. 24A is a perspective view of a retaining cap viewed from a bone screw retaining side in accordance with the principles of the present disclosure;
FIG. 24B is a perspective view of a retaining cap viewed from a bone screw retaining side in accordance with the principles of the present disclosure;
FIG. 25 is a side view of a gear mechanism in accordance with the principles of the present disclosure;
FIG. 26A is a top view of a gear mechanism in accordance with the principles of the present disclosure;
FIG. 26B is a bottom view of a gear mechanism in accordance with the principles of the present disclosure;
FIG. 27A is a perspective view of a first portion of a gear mechanism and a retaining cap in accordance with the principles of the present disclosure;
FIG. 27B is a perspective view of a first portion of a gear mechanism and a retaining cap in accordance with the principles of the present disclosure;
FIG. 28 is a cross section view of a tip portion of an example screwdriver in accordance with the principles of the present disclosure;
FIG. 29 is a removed parts view of a tip portion of an example screwdriver in accordance with the principles of the present disclosure;
FIG. 30 is a removed parts view of an example gear mechanism in accordance with the principles of the present disclosure;
FIG. 31 is a removed parts view of an alternate example gear mechanism in accordance with the principles of the present disclosure;
FIG. 32A is a side view of a tip portion of an example screwdriver in accordance with the principles of the present disclosure;
FIG. 32B is a side view of a tip portion of an example screwdriver with compression spring in accordance with the principles of the present disclosure; and
FIG. 33 is a perspective view of an example medical device that includes bone screw apertures that example screwdrivers of the present disclosure may progressively drive a bone screw through.

### DETAILED DESCRIPTION

As used herein, standard anatomical terms of location have their ordinary meaning as they would be understood by a person of ordinary skill in the art unless clearly defined or explained otherwise. It should be understood that various aspects disclosed herein may be combined in different combinations than the combinations specifically presented in the description and accompanying drawings. For example, characteristics of one embodiment may be combined or substituted with characteristics of another different embodiment unless those characteristics are clearly explained as being mutually exclusive. It should also be understood that, depending on the example, certain acts or events of any of the processes or methods described herein may be performed in a different sequence, may be added, merged, or left out altogether (e.g., all described acts or events may not be necessary to carry out the disclosed techniques and methods). In addition, while certain aspects of this disclosure are described as being performed by a single module, unit, or component for purposes of clarity, it should be understood that the techniques of this disclosure may be performed by a combination of units, modules, or components associated with, for example, a medical device such as a drill.

In some embodiments, the present disclosure is directed to surgical tools having a movable handle. For example, a surgical tool such as a drill and/or a screw driver having a movable handle that is optimized for a medical setting and can be readily used for various types of surgical techniques, including anterior surgical techniques, lateral surgical techniques, and oblique surgical techniques. In some embodiments, a surgical tool may be optimized to secure a spinal implant between adjacent vertebrae by securing at least one complimentary bone screw to the spinal implant and into an adjacent vertebrae. In some embodiments, and as mentioned above, the present disclosure may be employed in conjunction with spinal implants to treat spinal disorders such as, for example, degenerative disc disease, disc herniation, osteoporosis, spondylolisthesis, stenosis, scoliosis and other curvature abnormalities, kyphosis, tumor and fractures. In some embodiments, the present disclosure may be employed with other osteal and bone related applications, including those associated with diagnostics and therapeutics.

In some embodiments, the disclosed example surgical tools may be alternatively employed in a surgical treatment with a patient in a prone or supine position, and/or employ various surgical approaches to the spine, including anterior, posterior, posterior mid-line, direct lateral, postero-lateral oblique, and/or antero lateral oblique approaches, and in other body regions. The present disclosure may also be alternatively employed with procedures for treating the lumbar, cervical, thoracic, sacral and pelvic regions of a spinal column. The surgical tools of the present disclosure may also be used on animals, bone models and other non-living substrates, such as, for example, in training, testing and demonstration.

The present disclosure may be understood more readily by reference to the following detailed description of the embodiments taken in connection with the accompanying drawing figures, which form a part of this disclosure. It is to be understood that this application is not limited to the specific devices, methods, conditions or parameters described and/or shown herein, and that the terminology used herein is for the purpose of describing particular embodiments by way of example only and is not intended to be limiting. In some embodiments, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value and all numerical values therebetween. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior." Generally, similar spatial references of different aspects or components, *e.g.,* a "proximal end" of one component and a "proximal end" of a different component, indicate similar spatial orientation and/or positioning, *i*.*e*., that each "proximal end" is situated on or directed towards the same end of the device. Further, the use of various spatial terminology herein should not be interpreted to limit the various insertion techniques or orientations of the implant relative to the positions in the spine.

As used in the specification and including the appended claims, "treating" or "treatment" of a disease or condition refers to performing a procedure that may include administering one or more drugs, biologics, bone grafts (including allograft, autograft, xenograft, for example) or bone-growth promoting materials to a patient (human, normal or otherwise or other mammal), employing implantable devices, and/or employing instruments that treat the disease, such as, for example, micro-discectomy instruments used to remove portions bulging or herniated discs and/or bone spurs, in an effort to alleviate signs or symptoms of the disease or condition. Alleviation can occur prior to signs or symptoms of the disease or condition appearing, as well as after their appearance. Thus, treating or treatment includes preventing or prevention of disease or undesirable condition (*e.g*., preventing the disease from occurring in a patient, who may be predisposed to the disease but has not yet been diagnosed as having it). In addition, treating or treatment does not require complete alleviation of signs or symptoms, does not require a cure, and specifically includes procedures that have only a marginal effect on the patient. Treatment can include inhibiting the disease, *e.g.,* arresting its development, or relieving the disease, *e.g.,* causing regression of the disease. For example, treatment can include reducing acute or chronic inflammation; alleviating pain and mitigating and inducing re-growth of new ligament, bone and other tissues; as an adjunct in surgery; and/or any repair procedure. Also, as used in the specification and including the appended claims, the term "tissue" includes soft tissue, ligaments, tendons, cartilage and/or bone unless specifically referred to otherwise.

The components of disclosed embodiments described herein can be fabricated from biologically acceptable materials suitable for medical applications, including metals, synthetic polymers, ceramics and bone material and/or their composites. For example, the components of disclosed drills and bone screws, individually or collectively, can be fabricated from materials such as stainless steel alloys, commercially pure titanium, titanium alloys, Grade 5 titanium, super-elastic titanium alloys, cobalt-chrome alloys, stainless steel alloys, superelastic metallic alloys (*e.g.,* Nitinol, super elasto-plastic metals, such as GUM METAL^{®}), ceramics and composites thereof such as calcium phosphate (*e.g.,* SKELITE^{™}), thermoplastics such as polyaryletherketone (PAEK) including polyetheretherketone (PEEK), polyetherketoneketone (PEKK) and polyetherketone (PEK), carbon-PEEK composites, PEEK-BaSO4 polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaprolactone and their combinations.

Various components of disclosed embodiments may be formed or constructed of material composites, including but not limited to the above-described materials, to achieve various desired characteristics such as strength, rigidity, elasticity, compliance, biomechanical performance, durability and radiolucency or imaging preference. The components of expandable spinal implant system, individually or collectively, may also be fabricated from a heterogeneous material such as a combination of two or more of the above-described materials. The components of disclosed embodiments may be monolithically formed, integrally connected or include fastening elements and/or instruments, as described herein. For example, in some embodiments components comprising PEEK and/or titanium structures with radiolucent markers (such as tantalum pins and/or spikes) may be selectively placed on a drill, a drill bit, or a spinal implant, for example. In various embodiments, a drill is configured to bore into an adjacent vertebrae to provide a medical practitioner with a pilot hole or passageway for placement and/or sizing information to assist a surgeon with driving a a corresponding bone screw. The components of disclosed embodiments may be formed using a variety of subtractive and additive manufacturing techniques, including, but not limited to machining, milling, extruding, molding, 3D-printing, sintering, coating, vapor deposition, and laser/beam melting. Furthermore, various components of the expandable spinal implant system may be coated or treated with a variety of additives or coatings to improve biocompatibility, bone growth promotion or other features. For example, disclosed bone screws, may be selectively coated with bone growth promoting or bone ongrowth promoting surface treatments that may include, but are not limited to: titanium coatings (solid, porous or textured), hydroxyapatite coatings, or titanium plates (solid, porous or textured).

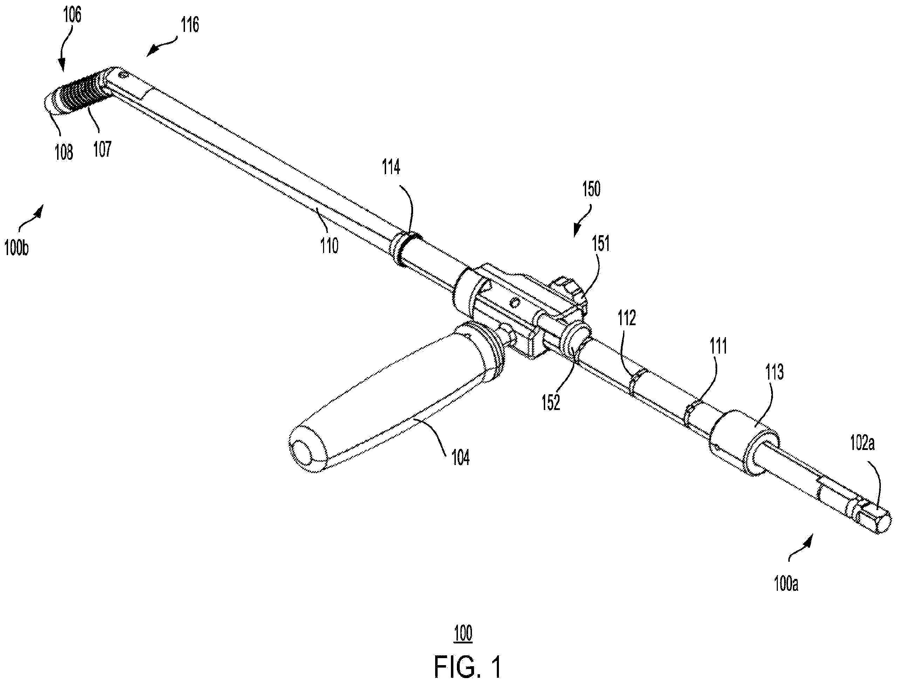

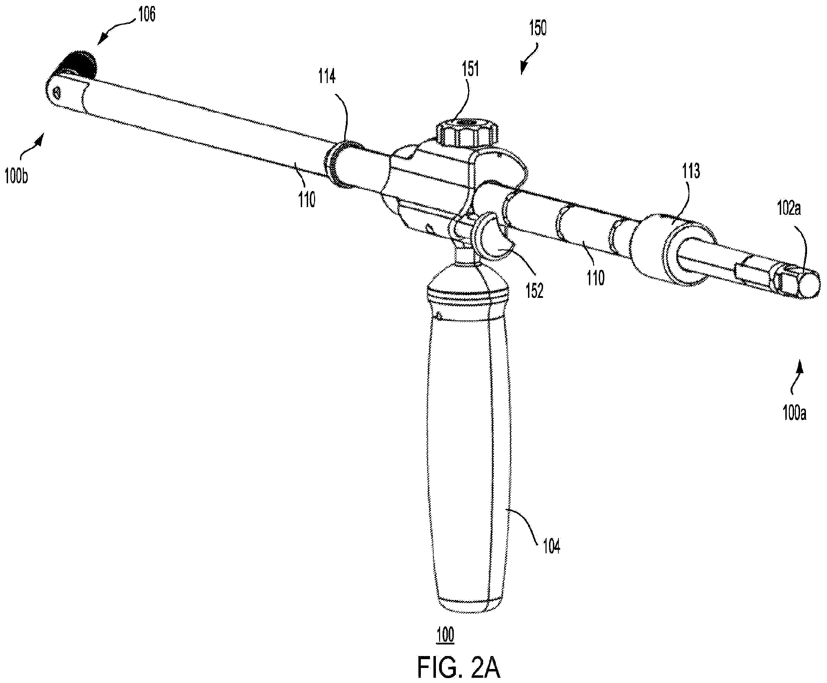

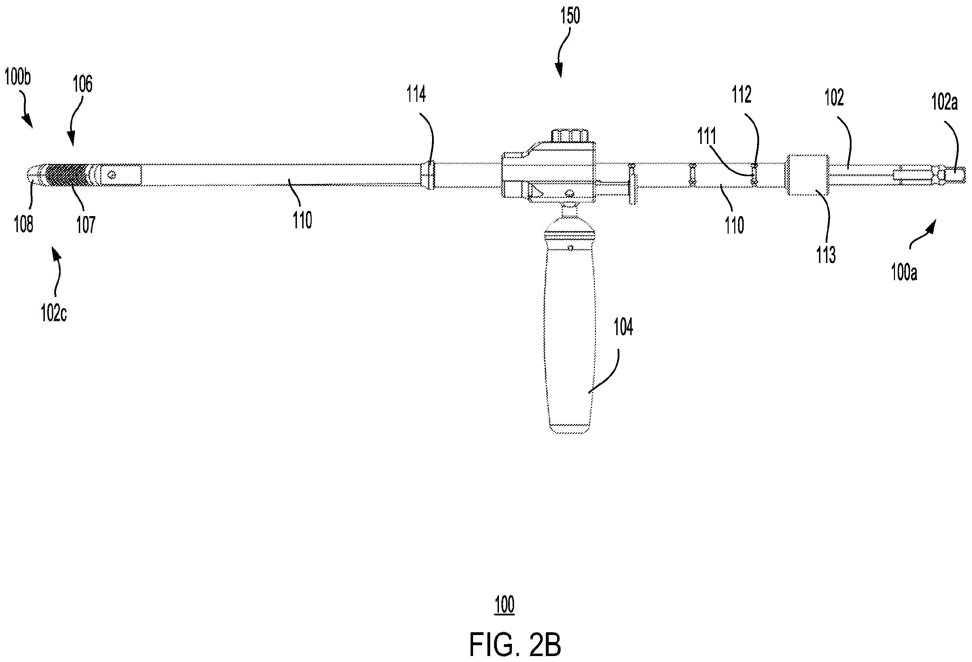

Referring generally to FIGS. 1-12 a first embodiment of an example surgical tool, e.g., a drill **100,** having a movable handle is illustrated. Referring generally to FIGS. 1-4B an example drill **100** is illustrated. FIG. 1 is a perspective view of an example drill **100,** FIG. 2A is an alternate perspective view of the example drill **100,** and FIG. 2B is a plan view of the example drill **100.** Drill **100** may include a proximal end **100a** and a distal end **100b.** Drill **100** may also include a drive shaft **102,** a positioning handle **104,** a tip portion **106,** a spring **107,** a sleeve **108,** a housing **110,** and a movable handle mechanism **150,** among other things.

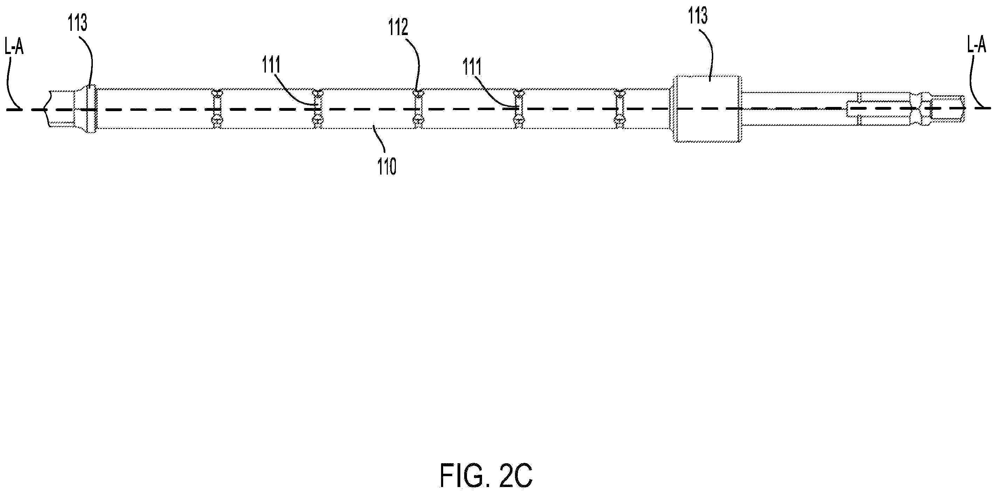

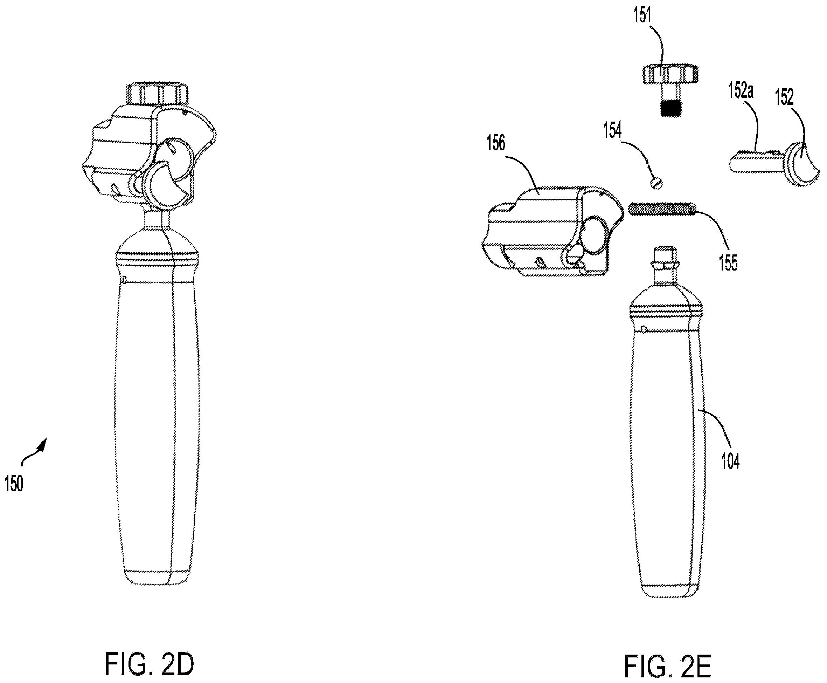

FIG. 2C is a top down view of a housing **110** of a surgical tool, FIG. 2D is a perspective view of an example movable handle mechanism **150,** and FIG. 2E is an exploded parts view of an example movable handle mechanism **150.** Housing **110** may define a longitudinal axis L-A and movable handle mechanism **150** may move forward and backward along housing **110** in a direction parallel to longitudinal axis L-A. Additionally, movable handle mechanism **150** may rotate around housing **110** and/or the longitudinal axis defined by housing **110,** for example. In various embodiments, movable handle mechanism **150** may move forward and backward in a longitudinal direction along the housing **110** between a proximal stop ring **113** and a distal stop ring **114,** for example. Movable handle mechanism **150** may also be rotatable about housing **110,** for example. In various embodiments, movable handle mechanism **150** may freely move forward and backward along housing **110** in a longitudinal direction and be securely coupled to housing **110** at any one channel **111** of a plurality of channels **111.**

In the illustrated embodiment, five channels **111** are shown although there may be more or less depending on the particular embodiment. Channels **111** may be spaced apart at any appropriate distance and need not be symmetrically spaced, for example. In at least one embodiment, channels **111** are symmetrically distributed between stop rings **113, 114** at a distance of about 25 mm, for example. Additionally, in various embodiments, movable handle mechanism **150** may freely rotate clockwise and/or counterclockwise around housing **110** and be securely coupled to housing **110** at any one detent **112** of a plurality of detents **112,** for example. In the illustrated embodiment, each channel **111** includes a plurality of symmetrically spaced and radially disposed detents **112** around the circumferential surface of housing **110,** for example. In various embodiments, there may be about 2-8 detents **112,** 4-6 detents **112,** and more particularly about 6 detents **112.**

FIG. 2D illustrates an example movable handle mechanism **150** for use with a multitude of surgical tools, for example. FIG. 2E illustrates example components of movable handle mechanism **150.** Movable handle mechanism **150** may include a body portion **156** including a longitudinally extending aperture therein which housing **110** may extend through, for example. In turn, positioning handle **104** may be coupled to body portion **156** at an underside thereof. Body portion **156** may include a cavity for receiving actuator **152,** for example. Actuator **152** may be referred to as a positioning actuator and take various forms. In the illustrated embodiment, actuator **152** comprises a spring loaded push button, for example. In operation, an end user may depress actuator **152** and thereby unseat positioning ball **154** from a corresponding detent **112.** After releasing actuator **152** spring **155** may bias positioning ball **154** against housing **110** to seat positioning ball **154** in any one of the plurality of detents **112,** for example. In various embodiments, actuator **152** includes ramped surfaces **152a** which facilitate seating positioning ball **154** as explained above.

Movable handle may also include at least one locking actuator configured to securely and rigidly couple movable handle mechanism **150** to housing **110,** for example. In the illustrated embodiment, locking actuator **151** comprises a rotatable knob configured to frictionally engage housing **110** by moving forward and backward in a direction substantially perpendicular to the longitudinal axis, for example. In various embodiments (not illustrated), locking actuator **151** may include a tip portion that may be seated within a corresponding detent **112** and/or channel **111,** for example. The tip portion may have a size and shape generally corresponding to a size and shape of a corresponding detent **112** and/or channel **111,** for example. Consistent with the disclosure herein, the movable handle mechanism **150** may be securely positioned at various locations forward and backward along housing **110** in a longitudinal direction and rotate clockwise and counterclockwise about the housing **110.** At least one advantage of this configuration is that an end user may position the positioning handle **104** in a location to absorb and/or counter the torque generated at the angled tip portion **106.** For example, the positioning handle can be located at a depth and a rotational position counter to the axis of rotation of the driver to enable the end user the ability to effectively resist the torque generated at the angle tip portion **106** and prevent "walking."

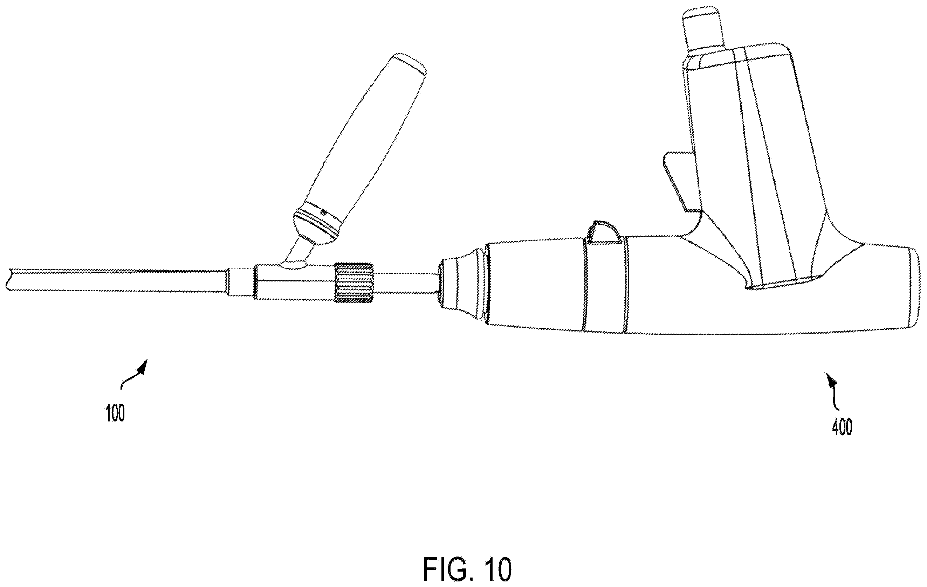

Drive shaft **102** may be configured to connect and disconnect with various types of drivers including manually operated handles and mechanically powered drive means that may be of a ratcheting or non-ratcheting type and which will be discussed in further detail below (*see, e.g.,* FIGS. 10-11). For example, drive shaft **102** may include a drive end **102a** and a main drive shaft portion **102b** extending in a longitudinal direction through a housing **110.** Drive end **102a** may comprise a variety of drive interfaces for coupling and uncoupling with various manually operated ratcheting handles and powered drivers, for example. Drive shaft **102** may freely rotate inside of housing **110** to transfer rotational force applied at the drive end **102a** at proximal end **100a** to drive end **102c** at distal end **100b,** for example. Positioning handle **104** may be securely held in place while drive shaft **102** freely rotates within housing **110.** Positioning handle **104** may be configured to assist with maintaining and controlling the drill **100,** *e.g.,* in view of torque transmitted through drive shaft and the corresponding resultant return forces. At least one advantage of having positioning handle **104** coupled to a movable handle mechanism **150** is that a surgeon may have greater freedom in movement and control maintaining drill **100** in a desired position while drilling a passageway (*e.g*., a pilot hole) for a bone screw **300.** For example, when drilling a passageway for a bone screw into the anatomy of a patient a return force may apply a rotational force against the drill **100** and a surgeon may be able to maintain the drill **100** in the desired position, for example. Additionally, in being able to move positioning handle **104** via movable handle mechanism **150** and drill **100** may be utilized for performing a wide variety of surgeries and also accounting for a greater variability in body types and ergonomic preferences of end users, for example.

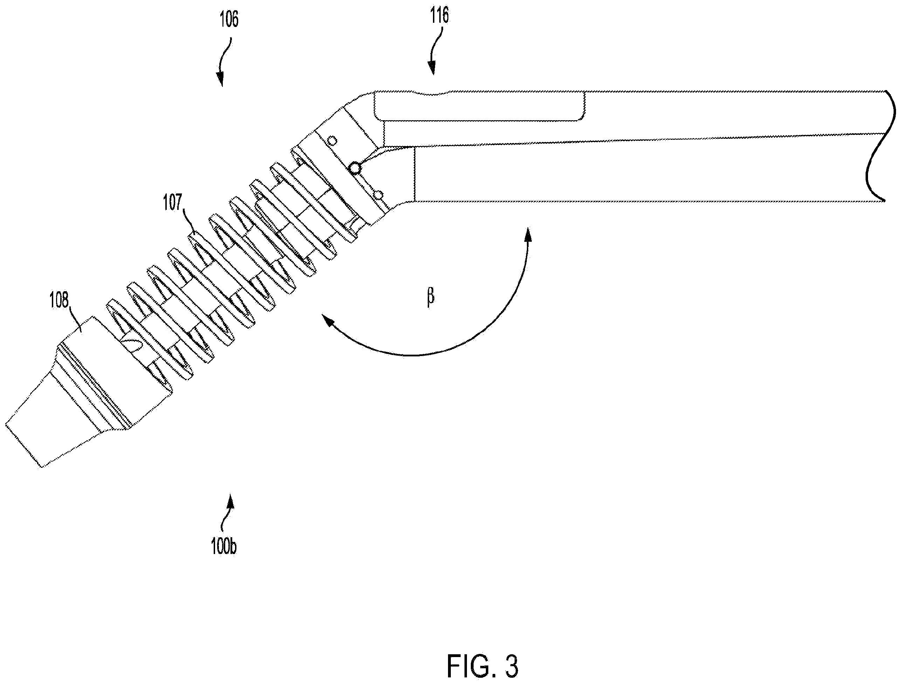

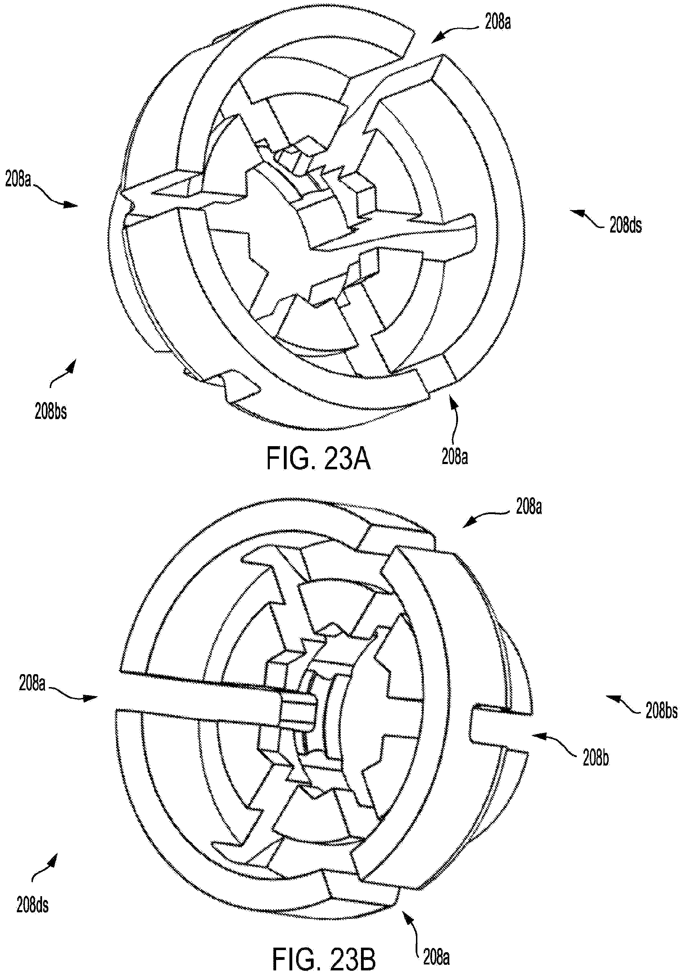

FIG. 3 is a magnified view of the tip portion **106** of an example drill **100** in accordance with the principles of the present disclosure. Tip portion **106** may be angled at a degree β (Beta) with respect to a longitudinal direction of housing **110** and/or drive shaft **102.** In various embodiments, tip portion **106** may extend in a direction that defines a drilling axis (rotation axis) of drill bit **109.** In some embodiments, tip portion **106** is angled such that the degree β corresponds to the ultimate desired angle of a passageway for receiving a bone screw **300.** In some embodiments, the degree β corresponds to an inclination of a bone screw aperture **1001** of a medical plate or medical device, *e.g*., medical device **1000** is a spinal implant including at least one bone screw aperture **1001** (*see* FIG. 23). In various embodiments, the tip portion **106** is angled to facilitate drilling of a passageway through a void space of a bone screw aperture **1001** of a spinal implant from a posterior approach while a patient is lying in a prone position, for example. Other surgical approaches, such as anterior, lateral, and/or posterior lateral approaches are also contemplated and may comprise adjustments to the degree β (Beta).

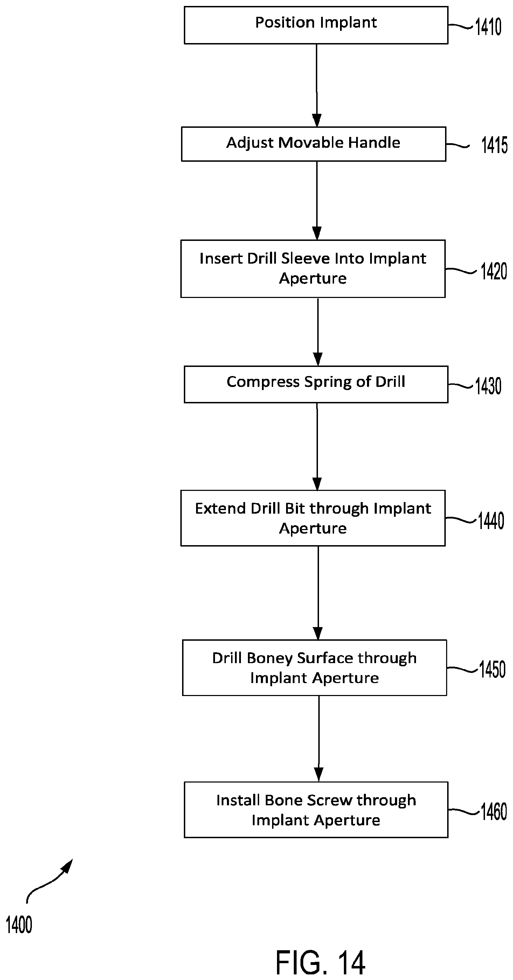

In various embodiments, tip portion **106** may be inclined about 20° - 60°, more particularly about 30° - 50°, and even more particularly about 40° - 45°, with respect to a longitudinal direction of housing **110.** However, it shall be understood that tip portion **106** may be angled at any degree β. Similarly, bone screw apertures **1001** may be angled at any degree with respect to endplates **1010, 1020** and tip portion **106** may be angled at a corresponding degree β to facilitate the installation of bone screw **300** therein (*see* FIG. 14). This angled arrangement may be advantageous for driving bone screw **300** while medical device **1000** is positioned between adjacent vertebral bodies. Furthermore, this angled arrangement may be advantageous to avoid anatomical landmarks and features such as the pelvic ring, rib cage, and iliac crest, of a patient, for example.

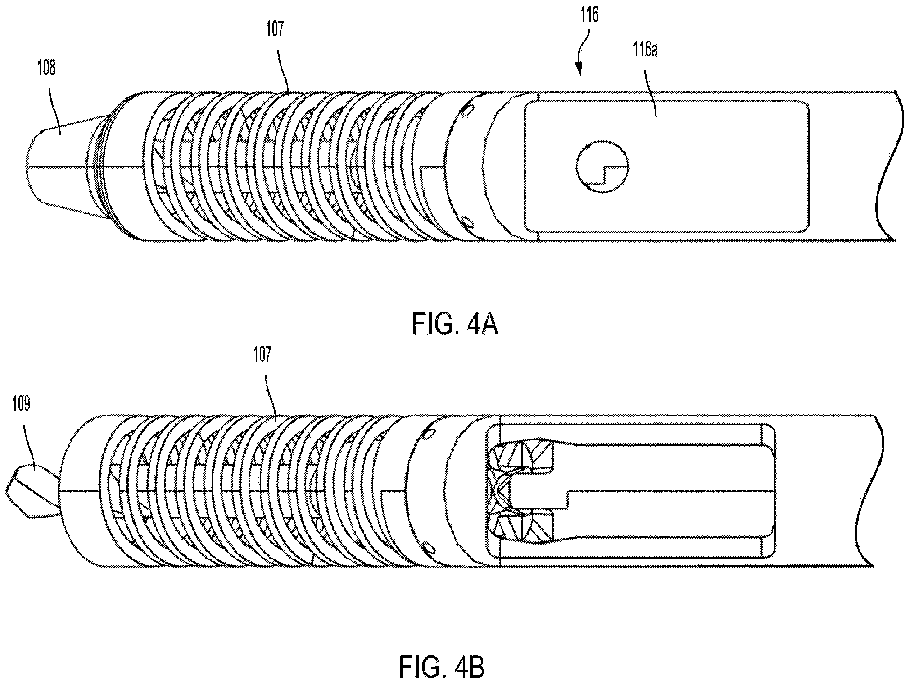

FIGS. 4A and 4B illustrate an example drill **100** that may include a flushing hole **116** having a flushing path to clean, lubricate, and/or inspect the components of tip portion **106.** For example, as shown in FIG. 4A a flushing hole **116** is shown, and in FIG. 4B a cover **116a** is removed to illustrate the flushing path. Flushing hole **116** may be advantageous for cleaning the interior orifices of tip portion **106.** Also shown in FIG. 4A is sleeve **108** which is a protective sleeve having a conical shape that surrounds drill bit **109.** For example, sleeve **108** may cover or surround drill bit **109** such that adjacent patient tissue is protected from drill bit **109.**

Sleeve **108** may be composed of elastomeric materials, thermoplastic materials, metallic materials, and various combinations thereof. In one embodiment, sleeve **108** is composed of an elastomeric material to provide flexibility and a high coefficient of friction for engaging and/or being seated within a bone screw aperture **1001,** for example. In an alternate embodiment, sleeve **108** is composed of metallic material, *e.g*., stainless steel and/or titanium. In another embodiment, sleeve **108** is composed of thermoplastic material, *e.g*., Polyether ether ketone (PEEK) and/or other organic thermoplastic polymers in, *e.g.,* the polyaryletherketone (PAEK) family. In another embodiment, sleeve **108** is composed of polyphenylsulfone (PPSU), also referred to as Radel by those with skill in the art. In another embodiment, sleeve **108** is composed of various combinations of the above enumerated materials. However, it shall be understood that the above enumerated materials are examples, and they shall not be construed as limiting.

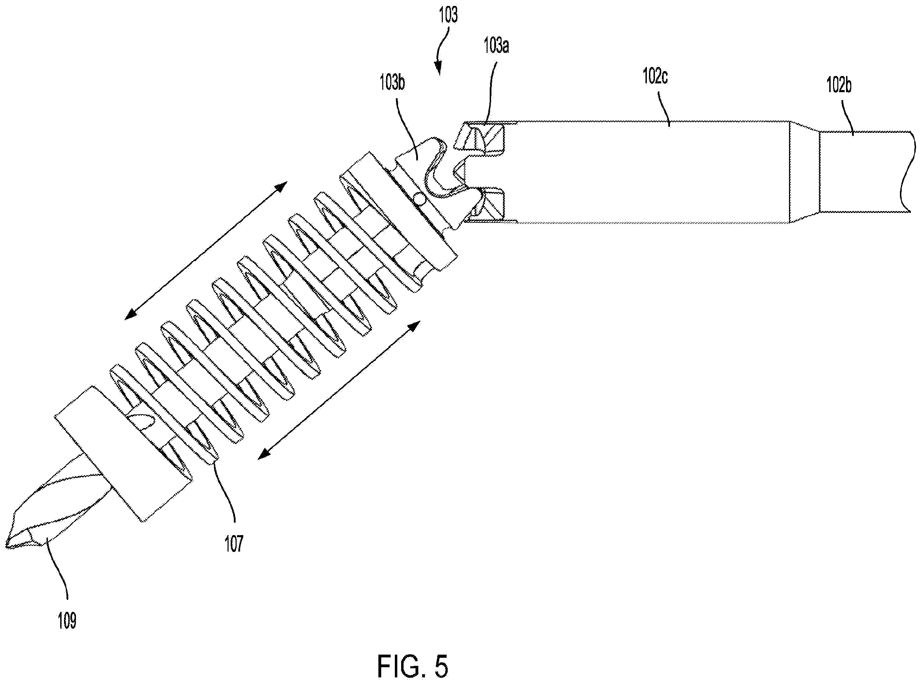

Referring generally to FIGS. 5-6B, operative characteristics of an example drill 100 will be explained. FIG. 5 illustrates a tip portion **106** (also referred to as an angled tip region) with a sleeve **108** and housing **110** removed for ease of explanation. In FIG. 5, it is shown that a spring **107** generally surrounds drill bit **109.** Spring **107** may be a compressible spring, a helical spring, a coil spring, of the like. In at least one embodiment, spring **107** takes the form of a compressible material such as rubber or foam. In some embodiments, spring **107** is covered by a protective cover to prevent debris and other foreign matter from entering in between the coils. In operation, a surgeon may press a proximal most end of sleeve **108** within a bone screw aperture **1001,** or alternatively against a surface to be drilled. In doing so, spring **107** may compress (shown by double sided arrows) and drill bit **109** may extend out of sleeve **108** (beyond sleeve **108**). For example, drill bit **109** is rigidly secured to drill **100** and sleeve **108** and spring **107** are movable with respect to drill bit **109.** At least one advantage of this arrangement, is that drill bit **109** may be protected and/or covered by sleeve **108** while drill bit **109** advances into a surface to be drilled and/or through bone screw aperture **1001.** For example, lateral sidewall surfaces of drill bit **109** are continuously protected from adjacent structures such as tissue while drill bit **109** continues to advance through sleeve **108** and into a boney structure.

Consistent with the disclosure herein, drill **100** may be understood as operating in various modes of operation. For example, a protected mode of operation and a drilling mode of operation. For example still, in a first mode of operation where the spring **107** is in a neutral position (non-compressed position) the sleeve **108** and spring **107** cover and/or surround the lateral sidewalls of the drill bit **109,** for example. In a second mode of operation where the spring **107** is in a compressed or partially compressed position due to the sleeve **108** acting against a bearing or retaining surface, the spring **107** may be compressed in a direction parallel to an extension direction of drill bit **109,** for example. The extension direction of drill bit **109** may be coincident with a rotation axis of the drill bit **109** (drilling axis). Accordingly, in the second mode of operation, and due to the compression of spring **107,** drill bit **109** may move through the sleeve **108** thereby exposing a tip of the drill bit **109** for drilling, for example. Additionally, in various embodiments and in the first mode of operation, the sleeve **108** and compressible spring **107** completely surround the lateral sidewalls of the drill bit **109.** Furthermore, in various embodiments, and in the first mode of operation, a distal most end of the sleeve **108** extends distally farther than a distal most end of the drill bit **109.**

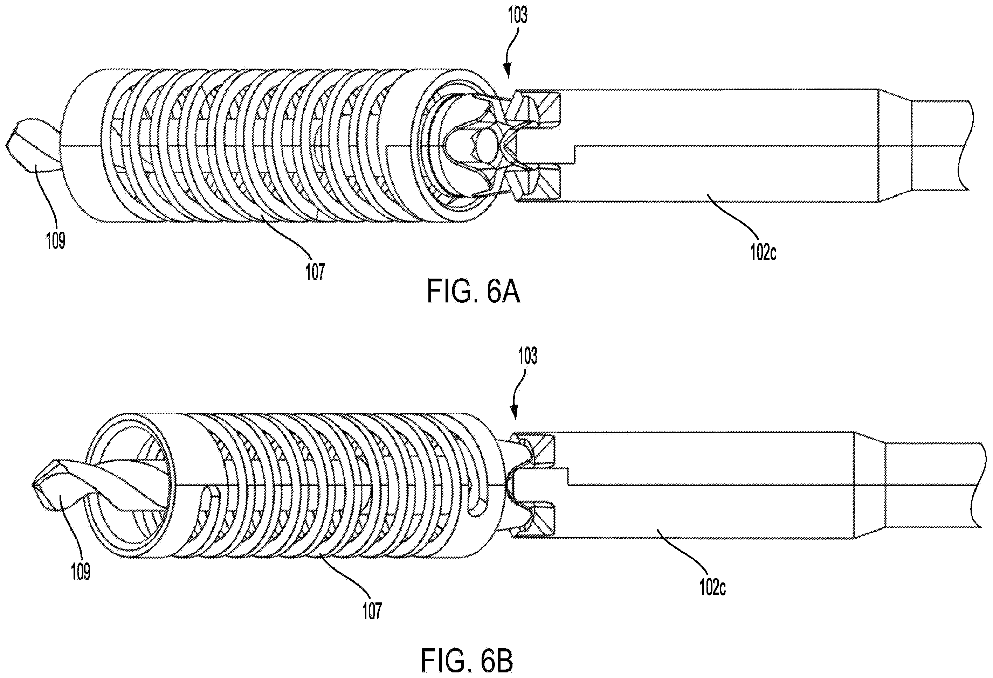

Also as shown in FIGS. 6-6b, an example gear mechanism **103** may be provided. Gear mechanism **103** may include worm gears, beveled gears, miter gears, planetary gears, sliding gears, helical or spiral gears, gear coupling parts, pawls, having teeth of various sizing and shapes for directing a rotation of the drive shaft **102** to drive end **102c.** For example, applying a rotation force at drive end **102a** may apply an equal or substantially equal rotation force at drill bit **109** because the gear mechanism **103** may redirect the rotation force. As illustrated, gear mechanism **103** may include a first body portion supporting a group of teeth **103a** that are meshed with a second group of teeth **103b** supported by a second body portion. In the example embodiment, the first group of teeth **103a** includes fourth teeth and the second group of teeth **103b** includes four teeth although the total number of teeth may be more or less. Those with skill in the art will readily appreciate that the particular geometry and number of teeth **103a, 103b** may be modified to accommodate any particular angle β (*see* FIG. 3). Additionally, in some embodiments, gear mechanism **103** may be designed to provide a mechanical advantage, such increasing or lowering the speed of rotation. For example, when a ratio of teeth sizing of teeth **103a, 103b** is inferior or superior with respect to the other.

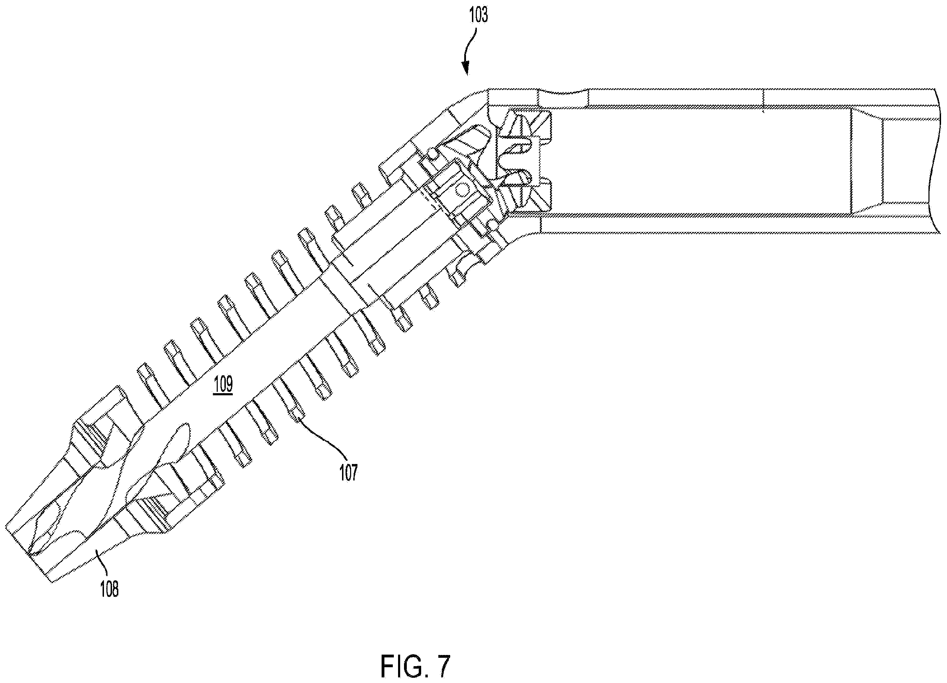

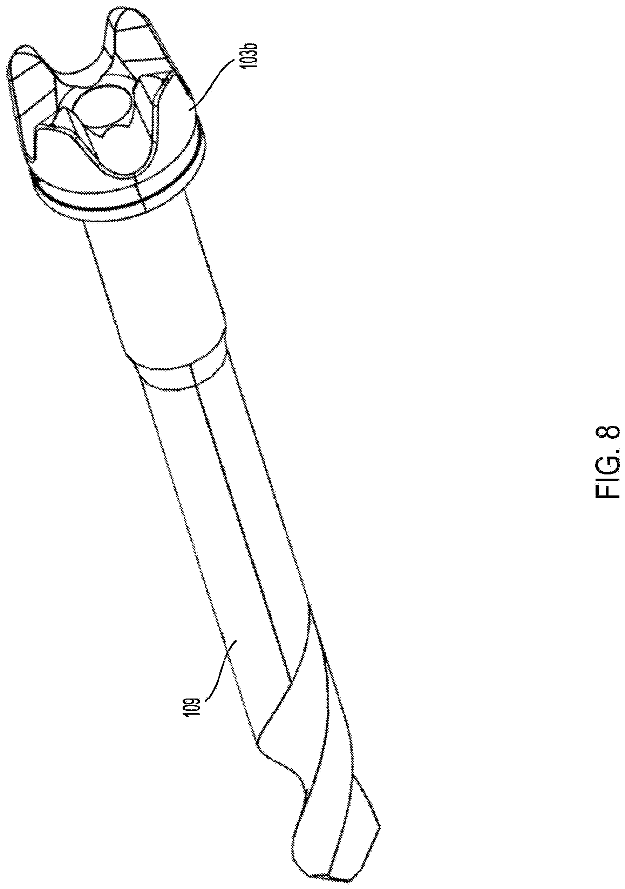

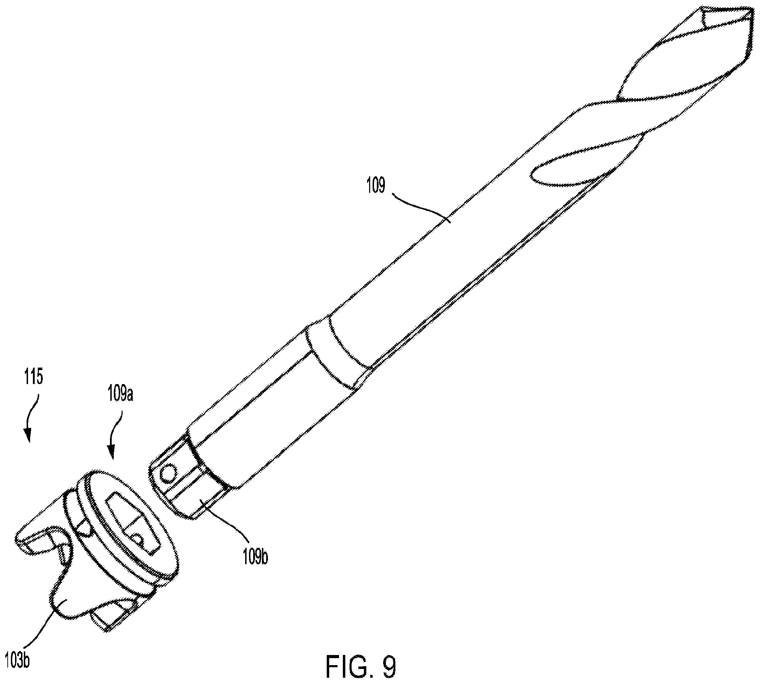

As illustrated in FIG. 7, a cross sectional view of spring **107,** sleeve **108,** drill bit **109,** and gear mechanism **103** is illustrated. In the cross sectional view, it is shown how drill bit **109** may rotate due to teeth **103a** being meshed with teeth **103b** while also being protected by spring **107** and sleeve **108.** As illustrated in FIG. 8, drill bit **109** may be coupled to an opposite side of the second body portion supporting teeth **103b,** for example. As illustrated in FIG. 9, a drill bit coupler **115** may comprise the second body portion and may include a drill bit aperture **109a** configured to receive a coupling end **109b** of drill bit **109** on one end and teeth **103b** on an opposite end. In some embodiments, the drill bit coupler **115** may include an extension shaft (not illustrated). The drill bit aperture **109a** and coupling end **109b** of drill bit **109** may correspond in size and shape to one another. For example, drill bit aperture **109a** and coupling end **109b** may have a hex, hexalobular, square, star, torx, prismoidal, polygonal, etc. shape dimensioned such that coupling end **109b** may be seated firmly within drill bit aperture **109a.**

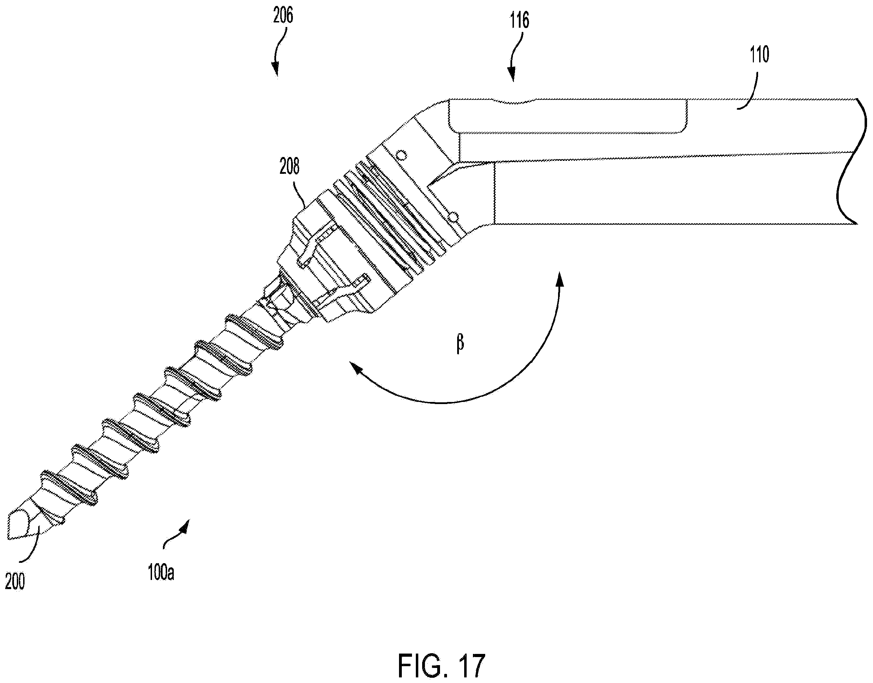

In an alternate embodiment, drill **100** may include a joint mechanism in lieu of gear mechanism **103** (not illustrated). For example, although not illustrated herein, the parent application from which this application is a continuation in part of, illustrates a joint mechanism **105** that may be substituted with gear mechanism **103.** For example, U.S. Pat. App. No. 17/123,906, discloses a joint mechanism **105** at FIG. 17 that may be operable/drivable via drive shaft **102** in the same, similar, or substantially the same way as gear mechanism **103** as explained above.

In an alternate embodiment, drill **100** may include a flexible shaft that may bend through the angled tip region in lieu of gear mechanism **103** and/or the joint mechanism as described above. For example, a flexible shaft mechanism may extend from the proximal end **100a** to the angled tip region **106** where a drill bit **109** may be coupled to a distal end of the flexible shaft. For example still, the flexible shaft mechanism may comprise a first drive end **102a** and a second drive end **102b** comprising a drill bit coupler **115** or the like.

The described flexible shaft mechanism can be formed of an elastomeric and/or metallic material for example. In embodiments including metallic materials the flexible shaft mechanism may comprise an undulating pattern of transverse cuts or seams across the width of the flexible shaft mechanism that form flexible indentations enabling the flexibility of the described flexible shaft. For example, an undulating dove tail pattern, c-shaped pattern, webbed pattern, etc. For example still, the flexible shaft may be formed with a plurality of successive and organized cuts making the shaft flexible laterally although still strong in tension and sufficient to apply rotational forces to drill bit **109** similarly as explained herein. In at least one embodiment, the flexible shaft mechanism can be made of an assembly of springs. Additionally, the flexible shaft mechanism may extend longitudinally through housing **110** of drill **100** until a region approximately corresponding with drill bit coupler **115** and may include a drill bit coupler **115** and/or a similar aperture for receiving a drill bit **109** such as aperture **109a,** for example.

FIG. 10 illustrates an example drill **100** operably coupled to a powered driver 400 in accordance with the principles of the present disclosure. Powered driver **400** may be powered by any means, *e.g*., electrically operated or pneumatically operated. At least one example powered drill is the POWEREASE^{™} System sold by Medtronic and/or the powered rotary-type handpiece described in U.S. Pat. No. 10,456,122.

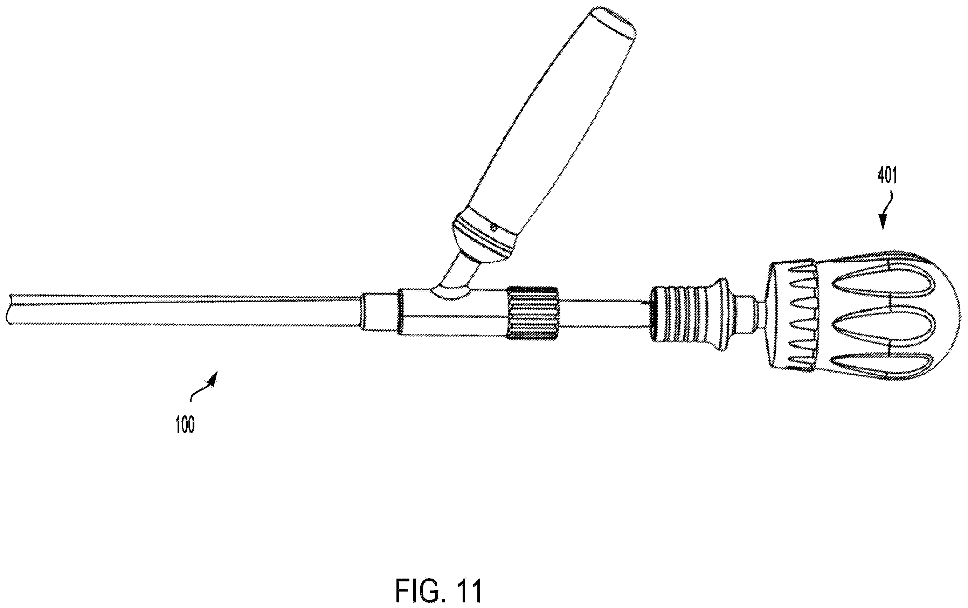

FIG. 11 illustrates an example drill **100** operably coupled to a manual hand driver **401** in accordance with the principles of the present disclosure. Hand driver **401** may selectively couple and uncouple with drive end **102a** of drive shaft **102.** At least one example of a manual hand driver **401** may be the commercially available QC handle sold by Medtronic of Minneapolis Minnesota. In various surgical techniques, a manual hand driver **401** as illustrated may be advantageous for performing gentle drilling, cleaning, excavation, and/or boring of a relatively soft or damaged bone, for example.

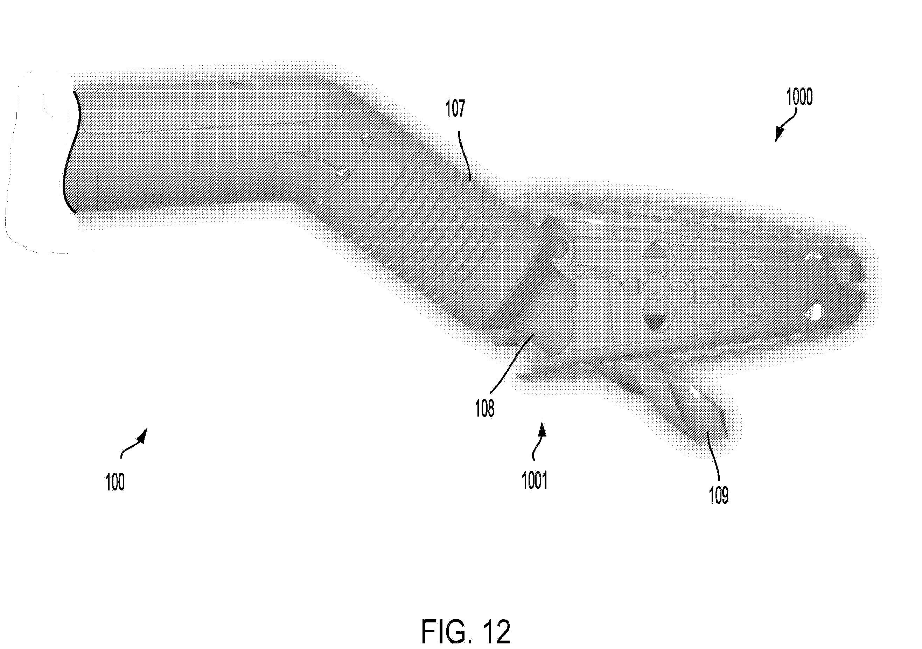

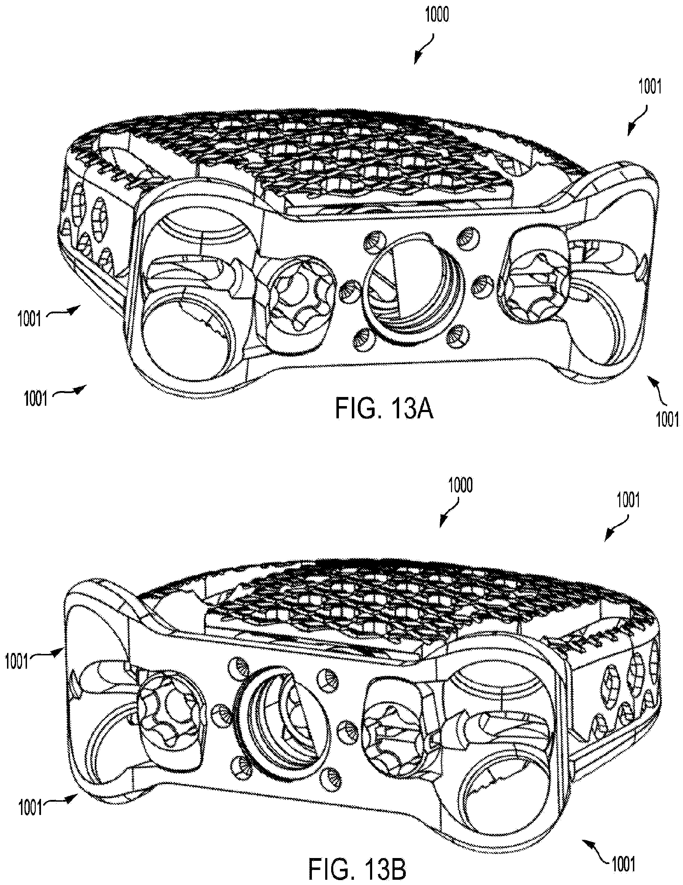

FIG. 12 illustrates an example drill **100** seated within a bone screw aperture **1001** of an implant **1000.** As illustrated, a conically shaped sleeve **108** is seated within a corresponding conically shaped bone screw aperture **1001** of implant **1000,** for example. For example, bone screw aperture **1001** may taper at the same or similar extend as sleeve **108** may taper and/or bone screw aperture **1001** may have a the same, similar, or substantially the same cross sectional dimensions. Drill **100** may be firmly pressed towards implant **1000** such that spring **107** is fully compressed thereby enabling drill bit **109** to extend through bone screw aperture **1001** beyond a bottom surface of a bottom endplate of implant **1000.** FIGS. 13A and 13B illustrate an alternate implant **1000** having four conically tapered bone screw apertures **1001.**

FIG. 14 illustrates an example method which does not form part of the invention as claimed. The method may be implemented with various drill **100** embodiments disclosed hereinabove, for example. In practice, at step **1410** an end user such as a surgeon may position an implant between adjacent vertebral bodies. The end user may expand the implant such that it is relatively firmly positioned between the two vertebral bodies (*e.g.,* a superior vertebral body and an inferior vertebral body). At step **1415,** the end user may adjust the movable handle longitudinally along the length of housing and/or rotationally around the housing, for example. In various embodiments, step **1415** may further comprise the substeps of (a) moving the movable handle assembly forward and/or backward in a longitudinal direction and (b) moving the movable handle assembly rotationally in a clockwise or counterclockwise direction around a longitudinally extending axis defined by the housing, for example. In various embodiments, moving the movable handle assembly forward/backward and/or clockwise/counterclockwise may further include the substep of seating a pin in a channel and/or seating a ball in a detent, for example. At step **1420,** the end user may be provided with a protected drill as disclosed herein and insert a conically tapered protective sleeve portion into a bone screw aperture of the implant. At step **1430,** the end user may firmly press the drill against the implant thereby compressing a protective spring of the drill. At step **1440,** and due in part to the compression performed at step **1430,** a drill bit may extend through the protective sleeve and through the implant aperture. Thereafter, at step **1450,** the end user may operably rotate a drive shaft of the drill and begin to drill a passageway into a boney surface, for example. In some embodiments, the bone screw aperture may be angled relative to the implant and/or surfaces of the adjacent boney structure. In those embodiments, due to the conically tapered sleeve portion being seated in an angled bone screw aperture, the passageway may be drilled at an angle that corresponds to the angle of the bone screw aperture. For example, the passageway and bone screw aperture may have the same, similar, or substantially the same angle as measured with respect to the implant and/or boney surface. At step **1460,** the end user may install a bone screw through the bone screw aperture and into the previously drilled passageway at a predefined angle. Furthermore, at step **1460** an end user may, for example, utilize the screwdriver **200** and coordinating bone screws as further described below.

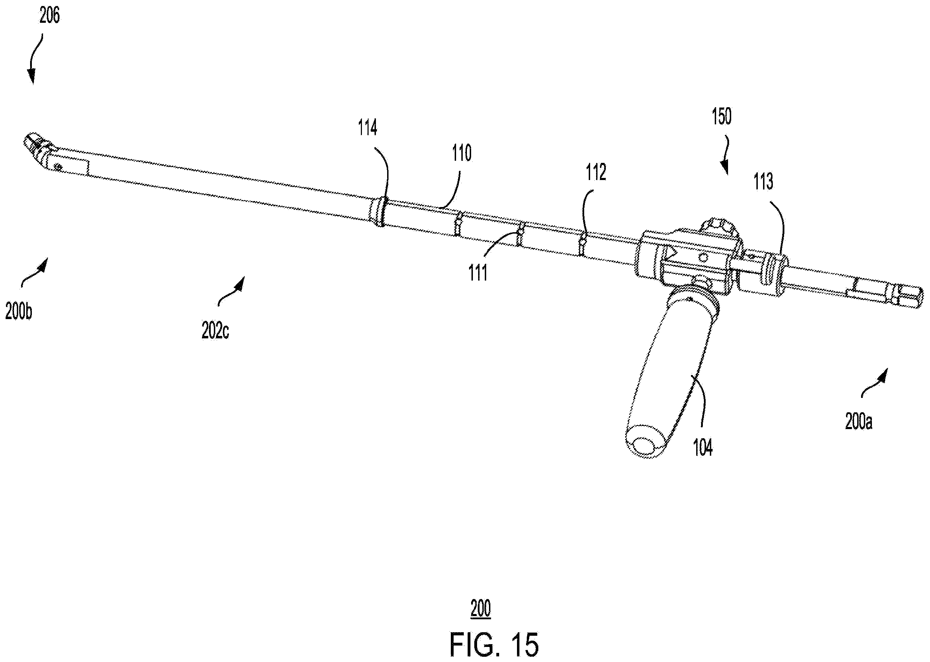

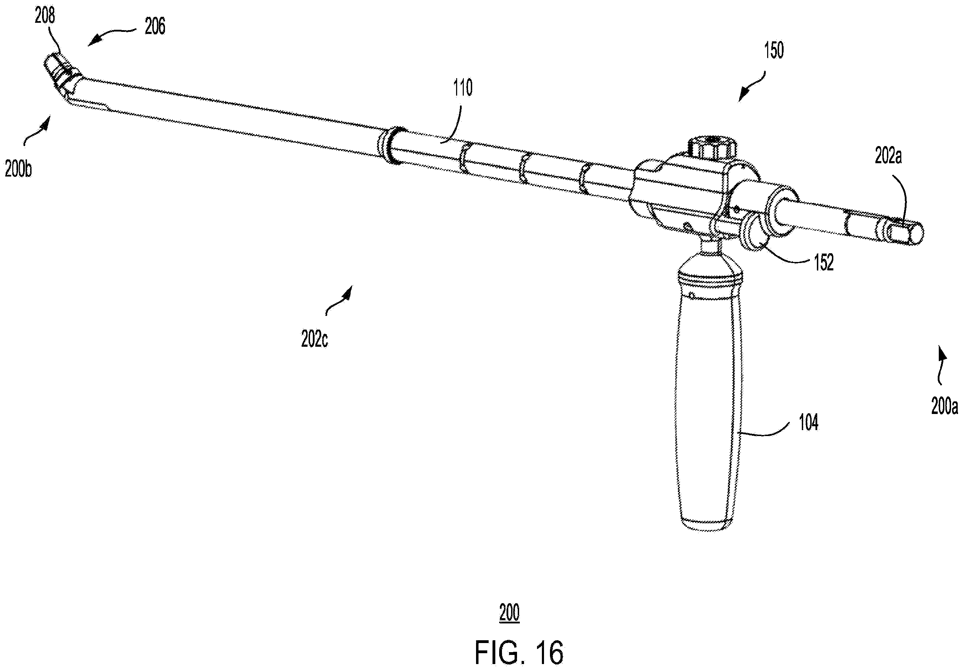

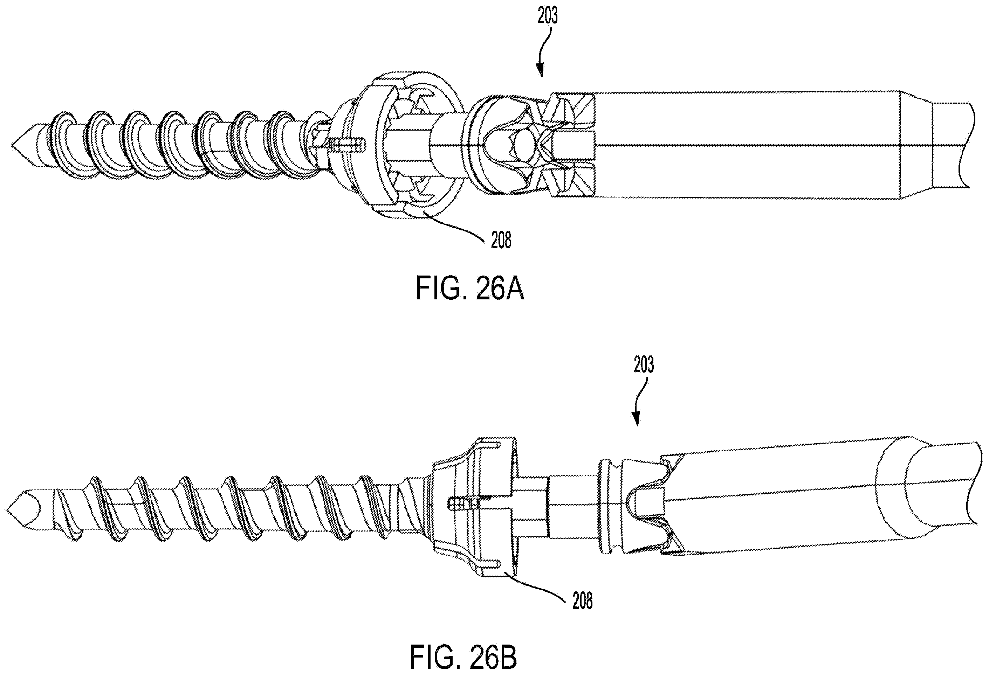

Referring generally to FIGS. 15-32B an example screwdriver **200** is illustrated. FIG. 15 is a perspective view of an example screwdriver **200** and FIG. 16 is a side view of the example screwdriver **200.** Screwdriver **200** may include a proximal end **200a** and a distal end **200b.** Screwdriver **200** may also include a drive shaft **202,** a positioning handle **104,** a movable handle mechanism **150,** a tip portion **206,** a retaining cap **208,** a movable handle mechanism **150,** and a housing **110,** among other things. Screwdriver **200** may include the same, similar, or substantially the same moving movable handle mechanism **150** as described above with respect to drill **100.** Additionally, drill **100** and screwdriver **200** may include the same, similar, or substantially the same components and functionality, where applicable, unless the context clearly indicates otherwise.

Housing **110** may define a longitudinal axis L-A (*see* FIG. 2C) and movable handle mechanism **150** may move forward and backward along housing **110** in a direction parallel to longitudinal axis L-A. Additionally, movable handle mechanism **150** may rotate around housing **110** and/or the longitudinal axis defined by housing **110,** for example. In various embodiments, movable handle mechanism **150** may move forward and backward in a longitudinal direction along the housing **110** between a proximal stop ring **113** and a distal stop ring **114,** for example. Movable handle mechanism **150** may also be rotatable about housing **110,** for example. In various embodiments, movable handle mechanism **150** may freely move forward and backward along housing **110** in a longitudinal direction and be securely coupled to housing **110** at any one channel **111** of a plurality of channels **111.**

In the illustrated embodiment, five channels **111** are shown although there may be more or less depending on the particular embodiment. Channels **111** may be spaced apart at any appropriate distance and need not be symmetrically spaced, for example. In at least one embodiment, channels **111** are symmetrically distributed between stop rings **113, 114** at a distance of about 25 mm, for example. Additionally, in various embodiments, movable handle mechanism **150** may freely rotate clockwise and/or counterclockwise around housing **110** and be securely coupled to housing **110** at any one detent **112** of a plurality of detents **112,** for example. In the illustrated embodiment, each channel **111** includes a plurality of symmetrically spaced and radially disposed detents **112** around the circumferential surface of housing **110,** for example. In various embodiments, there may be about 2-8 detents **112,** 4-6 detents **112,** and more particularly about 6 detents **112.**

As described above with respect to FIGS. 2D and FIG. 2E movable handle mechanism **150** may include a body portion **156** including a longitudinally extending aperture therein which housing **110** may extend through, for example. In turn, positioning handle **104** may be coupled to body portion **156** at an underside thereof. Body portion **156** may include a cavity for receiving actuator **152,** for example. Actuator **152** may be referred to as a positioning actuator and take various forms. In the illustrated embodiment, actuator **152** comprises a spring loaded push button, for example. In operation, an end user may depress actuator **152** and thereby unseat positioning ball **154** from a corresponding detent **112.** After releasing actuator **152** spring **155** may bias positioning ball **154** against housing **110** to seat positioning ball **154** in any one of the plurality of detents **112,** for example. In various embodiments, actuator **152** includes ramped surfaces **152a** which facilitate seating positioning ball **154** as explained above. Movable handle mechanism **150** may also include at least one locking actuator configured to securely and rigidly couple movable handle mechanism **150** to housing **110,** for example. In the example embodiment, locking actuator **151** comprises a rotatable knob configured to frictionally engage housing **110** by moving forward and backward in a direction substantially perpendicular to the longitudinal axis, for example. In various embodiments (not illustrated), locking actuator **151** may include a tip portion that may be seated within a corresponding detent **112** and/or channel **111,** for example. The tip portion may have a size and shape generally corresponding to a size and shape of a corresponding detent **112** and/or channel **111,** for example.

Referring back to FIGS. 15-28B drive shaft **202** may be configured to connect and disconnect with various types of drivers including manually operated handles and mechanically powered drive means that may be of a ratcheting or non-ratcheting type and which are discussed in further detail above (*see, e.g.,* FIGS. 10-11). For example, drive shaft **202** may include a drive portion **202a** disposed at a distal end thereof, a drive end **202b** disposed at a proximal end thereof, and a main drive shaft portion **202c** extending in a longitudinal direction through a housing **110.** Drive portion **202a** may comprise a variety of drive interfaces for coupling and uncoupling with various manually operated ratcheting handles and powered drivers. Drive shaft **202** may freely rotate inside of housing **110** to transfer rotational force applied at the drive portion **202a** to drive end **202b.** Positioning handle **104** may be securely held in place while drive shaft **202** freely rotates within housing **110.** Positioning handle **104** may be configured to assist with maintaining and controlling the screwdriver **200,** *e.g.,* in view of torque transmitted through drive shaft and the corresponding resultant return forces. At least one advantage of positioning handle **104** is that a surgeon may have greater control maintaining screwdriver **200** in a desired position while driving a bone screw **300.** For example, when installing a bone screw **300** into the anatomy of a patient a return force may apply a rotational force against the screwdriver **200** and a surgeon may be able to maintain the screwdriver **200** in the desired position.

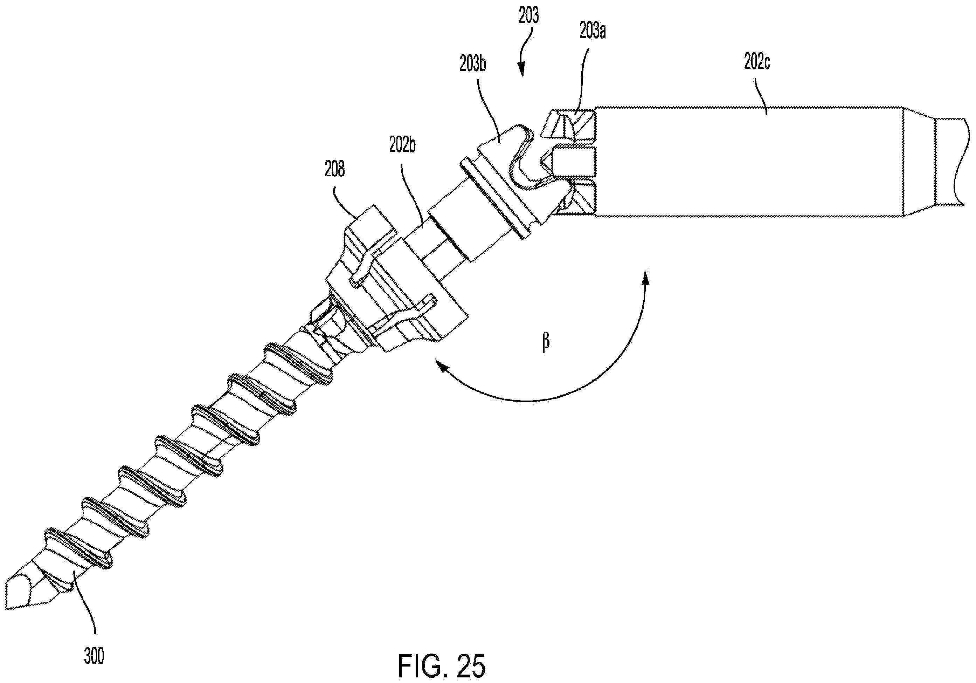

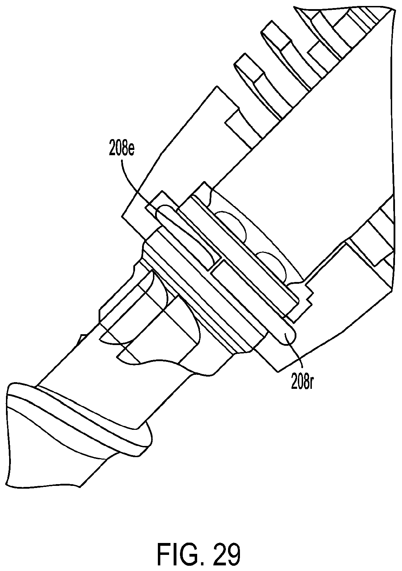

FIG. 17 is a magnified view of the tip portion **206** of an example screwdriver **200** in accordance with the principles of the present disclosure. Tip portion **206** may be angled at a degree β (Beta) with respect to a longitudinal direction of housing **110** and/or drive shaft portion **202c** (*see* FIG. 25). In some embodiments, tip portion **206** is angled such that the degree β corresponds to the desired inclination of bone screw **300.** In some embodiments, the degree β corresponds to an inclination of a bone screw aperture of a medical plate or medical device, *e.g*., medical device **1000** is a spinal implant including at least one bone screw aperture **1001** (*see* FIG. 29). For example, tip portion **206** may be inclined about 20° - 60°, more particularly about 30° - 50°, and even more particularly about 40° - 45°, with respect to a longitudinal direction of housing **110.** However, it shall be understood that tip portion **206** may be angled at any degree β. Similarly, bone screw apertures **1001** may be angled at any degree with respect to endplates **1010, 1020** and tip portion **206** may be angled at a corresponding degree β to facilitate the installation of bone screw **300** therein. This angled arrangement may be advantageous for driving bone screw **300** while medical device **1000** is positioned between adjacent vertebral bodies. Furthermore, this angled arrangement may be advantageous to avoid anatomical landmarks and features such as the pelvic ring, rib cage, and iliac crest, of a patient, for example.

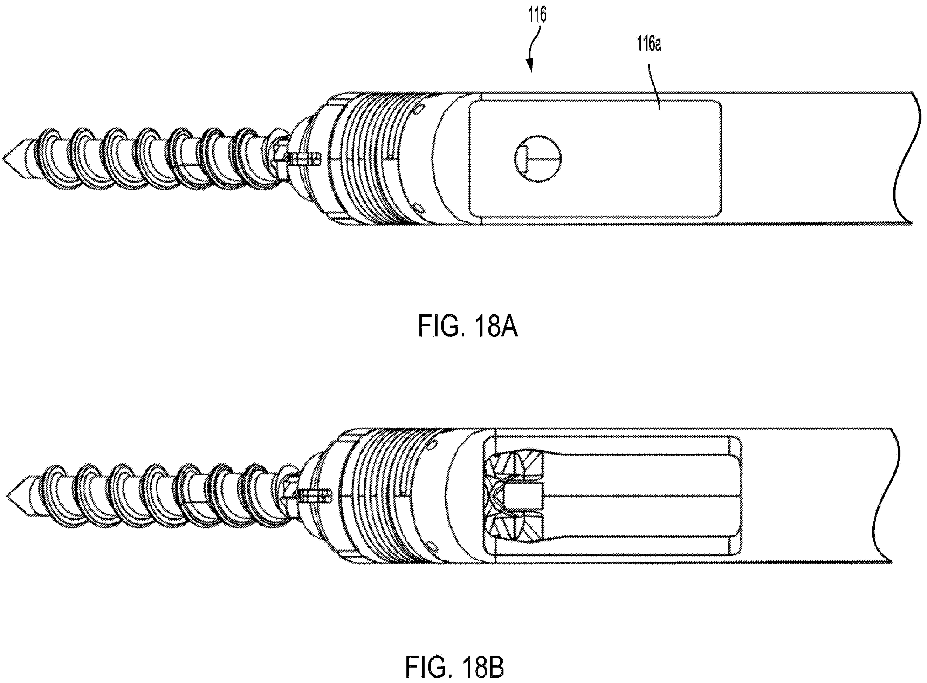

FIGS. 18A and 18B illustrate an example screwdriver **200** that may include a flushing hole **116** having a flushing path to clean the tip portion **206,** or to lubricate the mechanism. For example, as shown in FIG. 18A a flushing hole **116** is shown, and in FIG. 18B a cover **116a** is removed to illustrate the flushing path. Flushing hole **116** may be advantageous for cleaning the interior orifices of tip portion **206.**

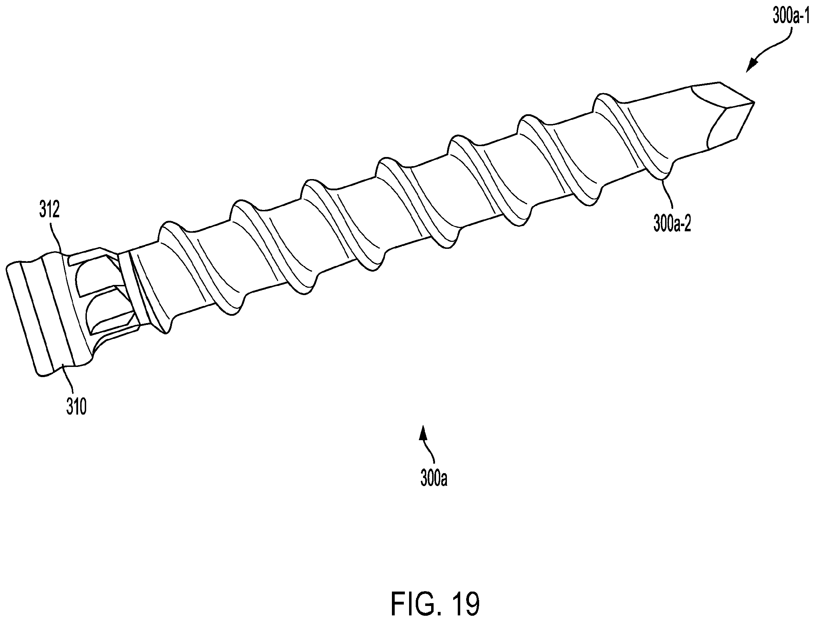

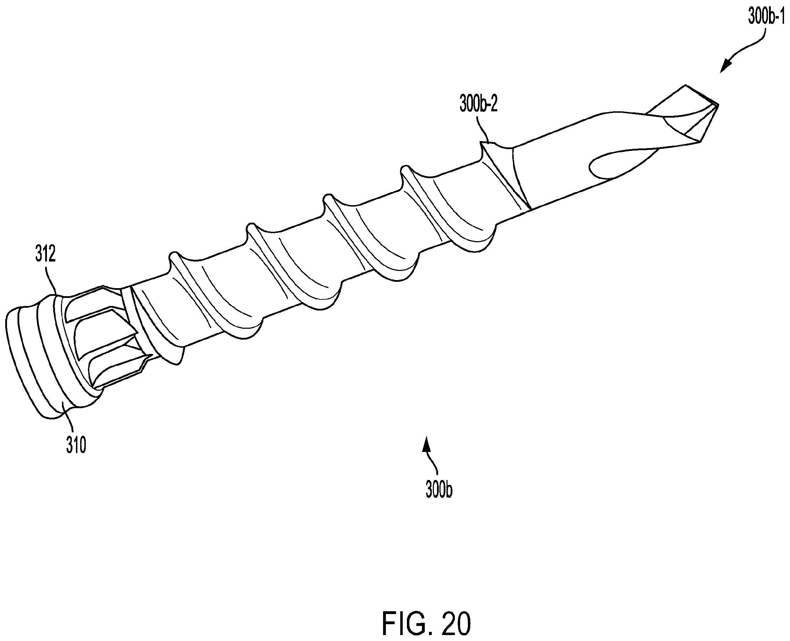

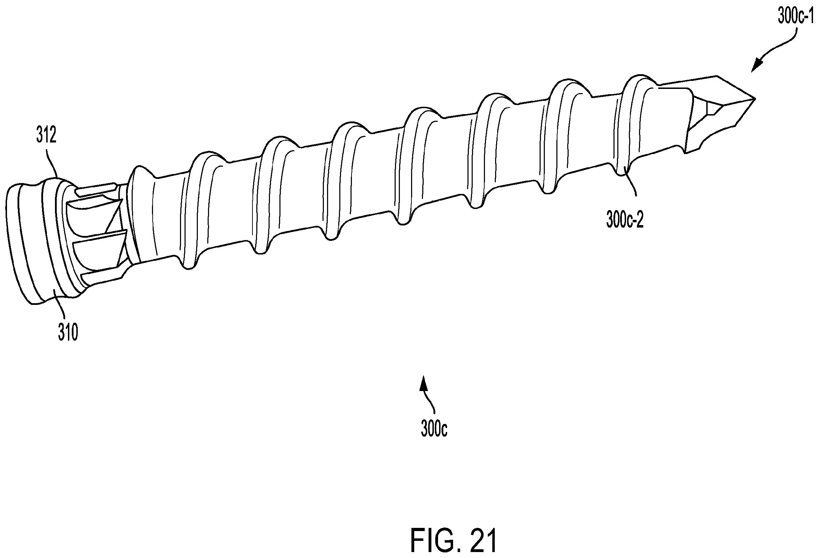

FIG. 19 is a perspective view of an example trocar tip bone screw **300a** suitable for use with disclosed embodiments in accordance with the principles of the present disclosure. FIG. 20 is a perspective view of an example flutes or fluted tip bone screw **300b** suitable for use with disclosed embodiments in accordance with the principles of the present disclosure. FIG. 21 is a perspective view of an example speed bone screw suitable for use with disclosed embodiments in accordance with the principles of the present disclosure. Each bone screw **300a, 300b, 300c** may be referred to throughout the disclosure generally as bone screw **300** to refer to bone screws generally and including any of the example bone screws **300a, 300b, 300c.** Additionally, each bone screw **300a, 300b, 300c** may have a single or multiple thread pitch and sizing that corresponds to a size of bone screw aperture **1001,** for example.

Trocar tip bone screw **300a** includes an angled tip portion **300a**-1 and a thread pattern including threads **300a**-2**.** Threads **300a**-2 may be spaced back from angled tip portion **300a**-1 which may facilitate with aligning bone screw **300a** with bone screw aperture **1001.** For example, in some embodiments, threads **300a**-2 are spaced back about 3 mm from angled tip portion **300a**-1. Fluted tip bone screw **300b** includes a cutting tip **300b**-1 and a thread pattern included threads **300b**-2. Cutting tip **300b**-1 may extend a relatively long distance from the beginning of threads **300b**-2 such that the cutting tip **300b**-1 may pre-drill into an anatomical feature or tissue such as a bone or an adjacent vertebral body before the threads **300b**-2 engage with bone screw aperture **1001.** For example, in some embodiments, threads **300b**-2 are spaced back about 8 mm from cutting tip **300b**-1. Speed bone screw **300c** includes a conical tip **300c**-1 and a thread pattern including threads **300c-**2. Different from trocar tip bone screw **300a** and fluted tip bone screw **300b,** threads **300c-**2 of speed bone screw **300c** may begin immediately adjacent conical tip **300c**-1.

Each of the example bone screws **300a, 300b, 300c** may be configured for use with example screwdrivers **200** disclosed herein. Additionally, each of the example bone screws **300a, 300b, 300c** may include an indent **310** spanning the circumference of the head portion and an adjacent edge portion **312** spanning the circumference of the head portion. In some embodiments, indent **310** may be defined by an arcuate circumferential groove that spans the circumference of a midsection of the head of bone screw **300.** Additionally, in some embodiments, edge portion **312** may be defined by an arcuate or chamfered edge spanning the circumference of the head portion adjacent the threads of bone screw **300.** The indent **310** and/or edge portion **312** may be advantageous for clipping and/or retaining bone screws **300a, 300b, 300c** in screwdriver **200,** as will be explained in more detail below.

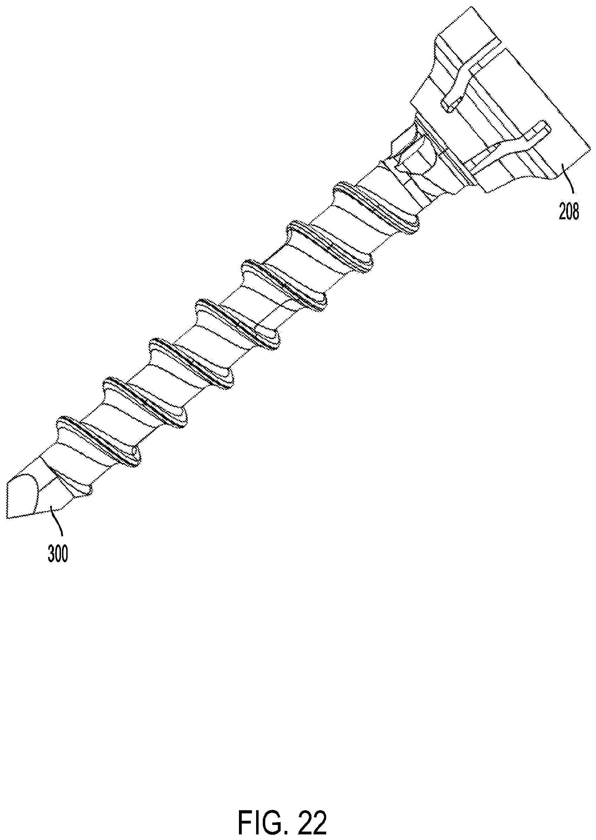

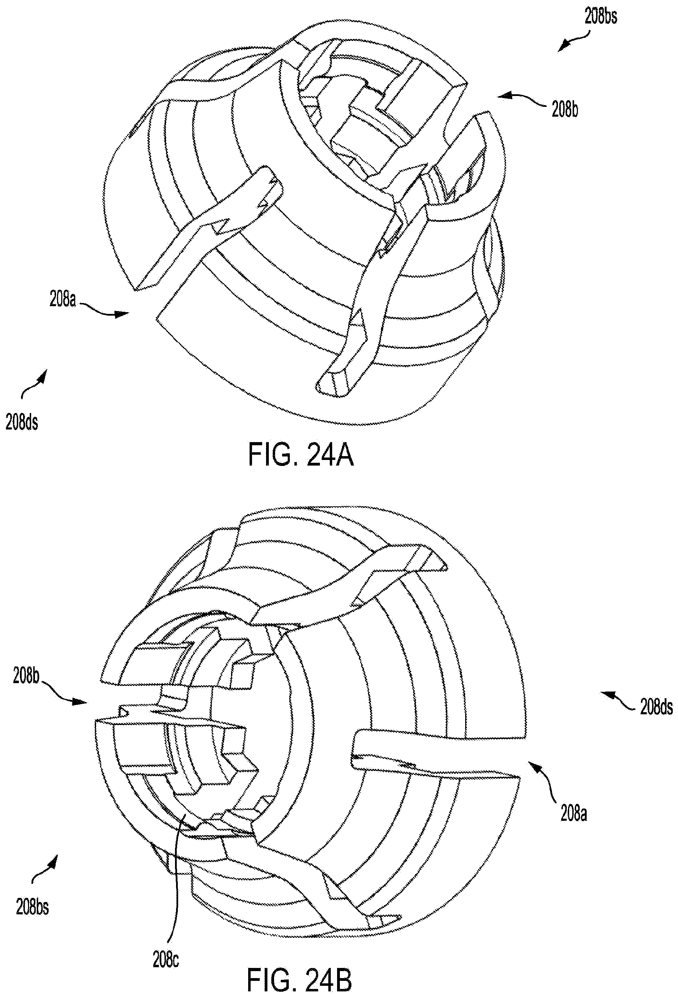

FIG. 22 is a perspective view of an example bone screw **300** coupled to and retained by an example retaining cap **208** in accordance with the principles of the present disclosure. FIGS. 23A and 23B are perspective views of retaining cap **208** viewed from a drive shaft connecting side **208ds** in accordance with the principles of the present disclosure and FIGS. 24A and 25B are perspective views of retaining cap **208** viewed from a bone screw connecting side **208bs** in accordance with the principles of the present disclosure. Retaining cap **208** may be composed of elastomeric materials, thermoplastic materials, metallic materials, and various combinations thereof. In one embodiment, retaining cap **208** is composed of metallic material, *e.g*., stainless steel and/or titanium. In another embodiment, retaining cap **208** is composed of elastomeric material, *e.g*., rubber and/or high-density rubber. In another embodiment, retaining cap **208** is composed of thermoplastic material, *e.g*., Polyether ether ketone (PEEK) and/or other organic thermoplastic polymers in, *e.g.,* the polyaryletherketone (PAEK) family. In another embodiment, retaining cap **208** is composed of polyphenylsulfone (PPSU), also referred to as Radel by those with skill in the art.

In the example embodiment, the drive shaft connecting side **208ds** includes a plurality of first aperture channels **208a** and the bone screw connecting side **208bs** includes a plurality of second aperture channels **208b.** In some embodiments, a circumference of the drive shaft connecting side **208ds** may be greater than a circumference of the bone screw connecting side **208bs.** First aperture channels **208a** may take the shape of a slit or narrow void extending from the drive shaft connecting side **208ds** towards the bone screw connecting side **208bs.** Second aperture channels **208b** may take the shape of a slit or narrow void extending from the drive shaft connecting side **208ds** towards the bone screw connecting side **208bs.** In the example embodiment, three (3) first aperture channels **208a** and three (3) second aperture channels **208b** are illustrated, although retaining cap **208** may have any number of aperture channels **208a, 208b.** For example, other embodiments may include two aperture channels **208a, 208b,** four aperture channels **208a, 208b,** or five aperture channels **208a, 208b,** for example. In the disclosed embodiment, first aperture channels **208a** are spaced apart symmetrically from one another around the circumference of the drive shaft connecting side **208ds.** Similarly, second aperture channels **208b** are spaced apart symmetrically from one another around the circumference of the bone screw connecting side **208bs.** Furthermore, each first aperture channel **208a** may be spaced between two immediately adjacent second aperture channels **208b** at a midpoint distance between the two immediately adjacent second aperture channels **208b.** Similarly, each second aperture channel **208b** may be spaced between two immediately adjacent first aperture channels **208a** at a midpoint distance between the two immediately adjacent first aperture channels **208a.** For example, as illustrated the aperture channels **208a, 208b** are symmetrically disposed around the retaining cap **208** on opposite sides thereof with respect to one another. Additionally, each aperture channel may extend from about 10%-95% of the length of retaining cap **208** and the length and width of each aperture channel may be adjusted to increase or decrease the desired flexibility of retaining cap **208.**

Additionally, retaining cap **208** may include a plurality of bumps **208c** at a bone screw connecting side **208bs.** Bumps **208c** may be inset from an outermost surface of the bone screw connecting side **208bs** on an interior thereof. Bumps **208c** may extend along the internal surface of retaining cap **208** at the bone screw connecting side **208bs.** In the disclosed embodiment, three bumps **208c** are illustrated although there may be more or less, for example two bumps **208c,** four bumps **208c,** or five bumps **208c.** In the disclosed embodiment, the number of bumps **208c** may correspond to the number of aperture channels **208b.** For example, in the disclosed embodiment there may be three aperture channels **208b** and three bumps **208c** where each bump is disposed symmetrically between adjacent aperture channels **208b.** Furthermore, a curved distance along the interior circumference of bone screw connecting side **208bs** between adjacent aperture channels **208b** may be greater than a curved distance (length) of bumps **208c.** For example, in some embodiments, a circumferential length of bumps **208c** may range from about 25% - 75% of the circumferential distance between adjacent aperture channels **208b.** Additionally, when viewed in cross section, bump **208c** may be shaped like an arc of a circle or cylinder. However, in other embodiments, bump **208c** may have alternate shapes such as prismoidal, polygonal, conical, etc. In the disclosed embodiment, bump **208c** may have a cross sectional shape generally corresponding to indent **310.** Accordingly, those with skill in the art will recognize that bump **208c** and indent **310** may take any shape and that it in some embodiments it may be advantageous that their shapes correspond with one another.

At least one advantage of the geometrical arrangement of aperture channels **208a, 208b** is that they may allow the retaining cap **208** to deform, at least partly, to couple/uncouple with the head of a bone screw **300** and/or drive end **202b.** For example, a bone screw **300** may be inserted into the retaining cap **208** and clipped or retained by the retaining cap **208** by inserting the head of the bone screw **300** into the retaining cap **208** with a sufficient force, *i.e.,* a clipping force. When inserting the head of the bone screw **300** into the retaining cap **208,** the retaining cap **208** may deform, at least partly, consistent with the above disclosure when clipping the bone screw **300.** In some embodiments, a clipping force may be about 1 N - 6 N (Newtons), more particularly about 2 N - 4 N, and even more particularly about 2.5 N. Similarly, the retaining cap **208** may be attached to drive end **202b.** For example, retaining cap **208** may be attached to drive end **202b** with an attachment force of about 10 N - 40 N, more particularly about 15 N - 30 N, and even more particularly about 20 N. In this way, retaining cap **208** may be considered elastic although not necessarily composed of an elastomeric material.

A particular advantage of a flexible retaining cap **208** as described herein is that it may (1) retain a bone screw **300** therein with a clipping force and (2) automatically release the bone screw **300** when it is sufficiently installed or anchored. For example, during an installation or driving procedure, a bone screw **300** may be retained in the retaining cap **208** until the bone screw **300** is sufficiently installed in a target object or surgical site such that a sufficient extraction force pulls the bone screw **300** from the retaining cap **208,** *i.e.,* the extraction force exceeds the clipping or retaining force. For example, when driving the bone screw **300** into the target object or surgical site, the retaining cap **208** may deform, at least partly, consistent with the above disclosure when releasing the bone screw **300.** In some embodiments, an extraction force may be about 2 N - 30 N, more particularly about 10 N - 20 N, and even more particularly about 15 N. In some embodiments, a ratio of the clipping force to the extraction force may about 1:15, more particularly about 1:10, and even more particularly about 1:6. For example, in at least one embodiment the retaining cap **208** is composed of a PEEK material and the clipping force is about 2.5 N and the extraction force is about 15 N.

Referring generally to FIGS. 25 - 27B, an example gear mechanism **203** may be provided. Gear mechanism **203** may include worm gears, beveled gears, miter gears, planetary gears, sliding gears, helical or spiral gears, gear coupling parts, pawls, having teeth of various sizing and shapes for directing a rotation of the drive shaft **202** to drive end **202b.** For example, applying a rotation force at drive portion **202a** may apply an equal or substantially equal rotation force at drive end **202b** because the gear mechanism **203** may redirect the rotation force. As illustrated, gear mechanism **203** may include a first group of teeth **203a** that are meshed with a second group of teeth **203b.** In the example embodiment, the first group of teeth **203a** includes fourth teeth and the second group of teeth **203b** includes four teeth although the total number of teeth may be more or less. Those with skill in the art will readily appreciate that the particular geometry and number of teeth **203a, 203b** may be modified to accommodate any particular angle β (*see* FIG. 25). Additionally, in some embodiments, gear mechanism **203** may be designed to provide a mechanical advantage, such increasing or lowering the speed of rotation. For example, when a ratio of teeth sizing of teeth **203a, 203b** is inferior or superior with respect to the other.

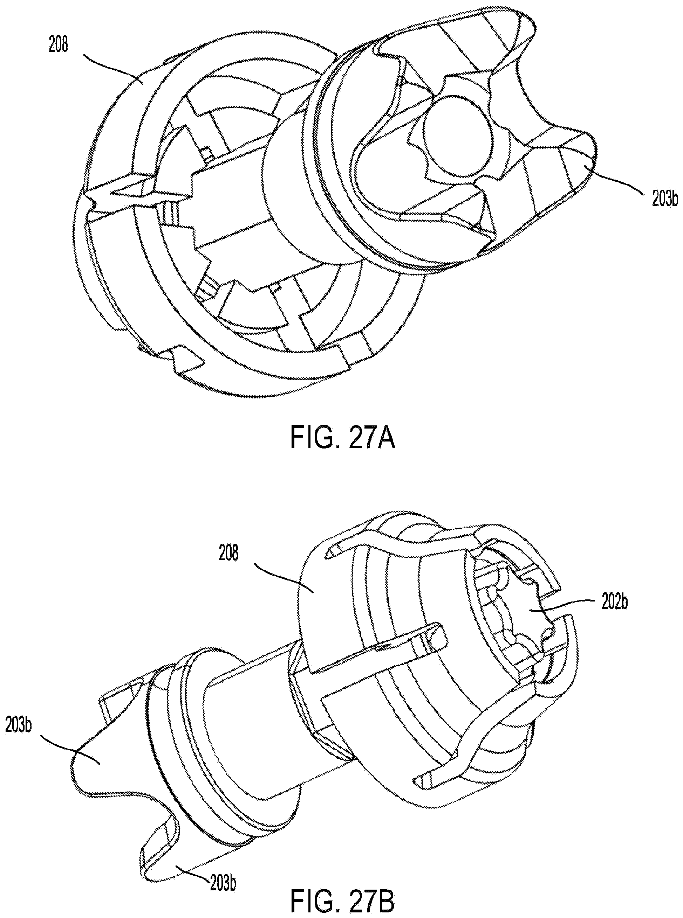

As illustrated best in FIG. 27B, drive end **202b** may disposed in an internal cavity of retaining cap **208** such that it may mate with a head of a corresponding bone screw **300.** Additionally, the retaining cap **208** may be coupled to drive end **202b** in such a way that it will also rotate when drive end **202b** rotates. In the illustrated embodiment, drive end **202b** includes a torx head driver configuration, although other designs are contemplated, including star or hexalobular configurations. For example, the drive end **202b** may resemble the geometry of the tip of a torx driver, hex driver, phillips driver, square head driver, hexalobular driver, polygonal driver, or the like. In at least one embodiment, a Torx T20 size driver may be used.

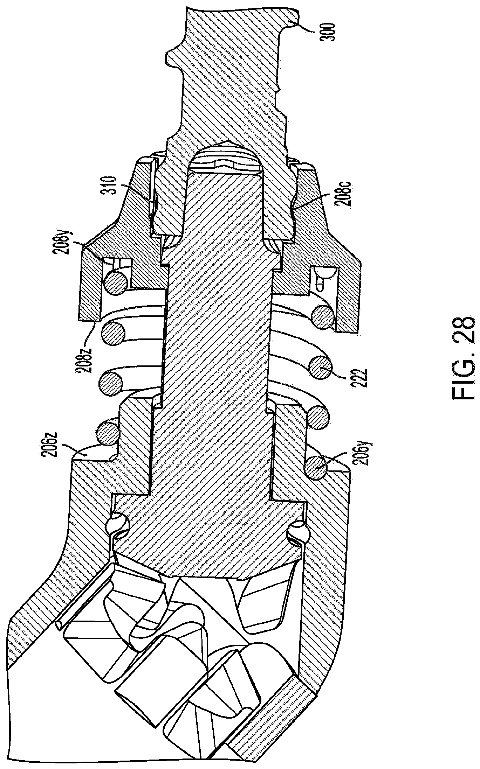

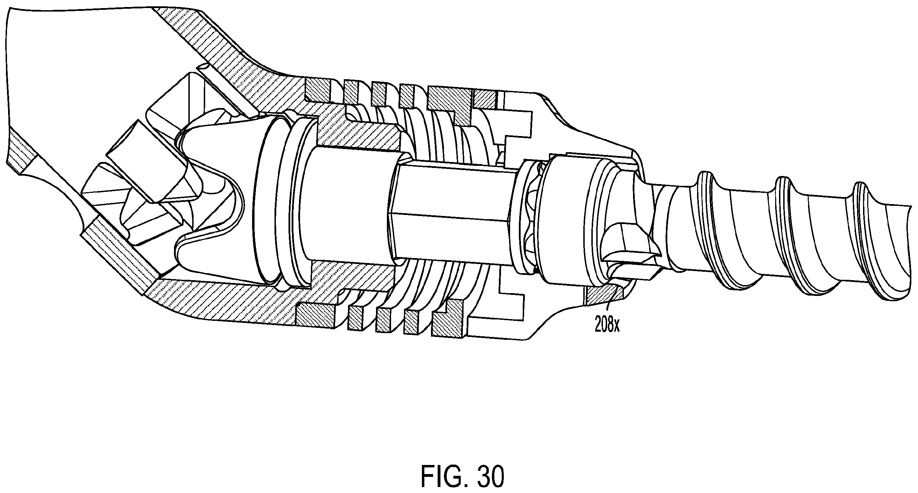

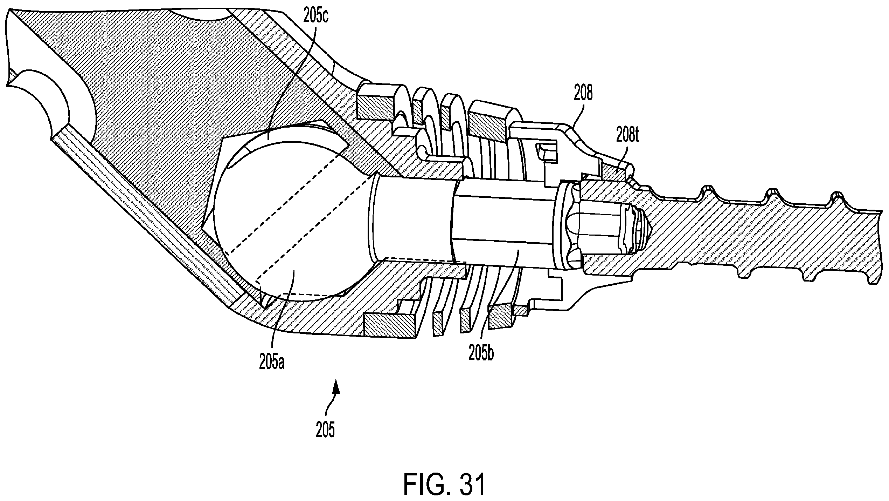

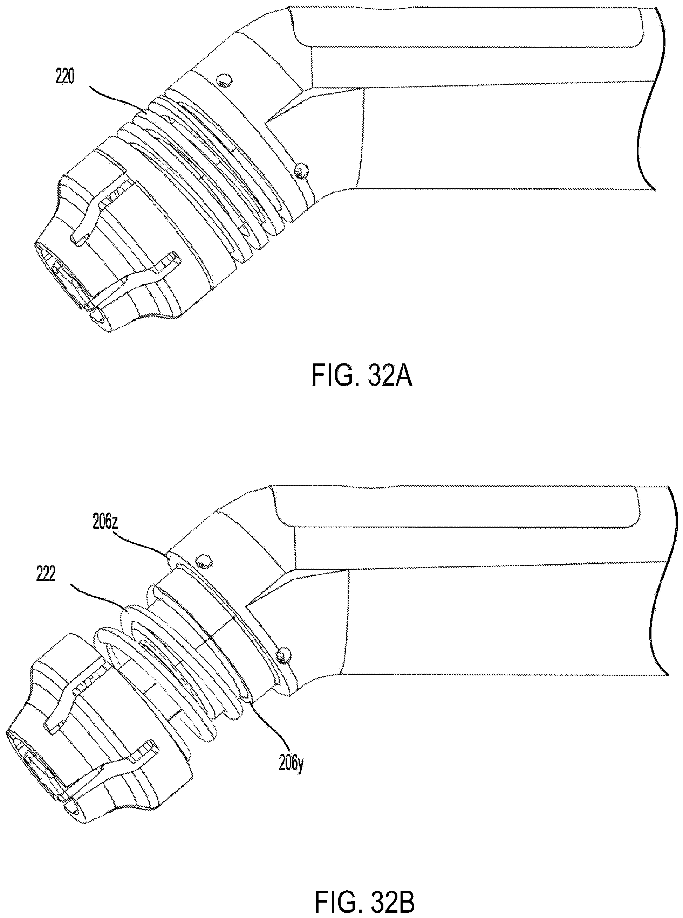

Referring generally to FIGS. 28 - 32B a tip portion **206** of an example screwdriver **200** may be illustrated. FIG. 28 is a cross section view of a tip portion **206** and FIG. 29 is a removed parts view of a tip portion **206** of an example screwdriver **200** in accordance with the principles of the present disclosure. FIG. 30 is a removed parts view of an example gear mechanism **203** in accordance with the principles of the present disclosure and FIG. 31 is a removed parts view of an alternate example gear mechanism **203** in accordance with the principles of the present disclosure. FIG. 32A is a side view of an example tip portion **206** in a fully assembled condition and FIG. 32B is a side view of an example tip portion **206** in accordance with the principles of the present disclosure.

In some embodiments, retaining cap **208** may include at least one retaining feature such as bump **208c,** for example. Additionally, some example bone screws **300** may include an indent **210.** In a head on view, the indent **210** may be defined by a radius of a circle that is slightly less than a radius defining a tip portion of the head of bone screw **300.** The retaining bump **208c** may have a curved surface profile including a width and depth that corresponds to the width and depth of the indent **210.** Additionally, when viewed in a cross sectional view, retaining bump **208c** may be seated within indent **210.** In disclosed embodiments, tip portion **206** may include a first spring **220** and a second spring **222.** However, in some embodiments, only one of first spring **220** or second spring **222** may be provided. Example springs **220, 222** may be referred to as industrial compression springs, machined springs, coil springs, and/or helical springs. First spring **220** may have outside surfaces that are flush with the adjacent surfaces, *i.e.,* outside surfaces of retaining cap **208** and tip portion **206** (*see* FIG. 32A). For example, first spring **220** may act against a bearing surface **208z** of retaining cap **208** and a bearing surface **206z** of tip portion **206** (*see* FIG. 28 and FIGS. 32A-32B). Second spring **222** may be disposed within a central cavity of first spring **220,** *i.e.,* second spring **222** may be surrounded by first spring **220.** For example, second spring **222** may act against corresponding inset radial surfaces of retaining cap **208** and tip portion **206** that are disposed and inset radially from the surfaces that first spring **220** acts against. For example, second spring **222** may act against channel **208y** of retaining cap **208** and bearing surface **206y** of tip portion **206.** In some embodiments, channel **208y** may extend circumferentially around an interior of retaining cap **208** that is radially inset from the outside surface of retaining cap **208.** Additionally, second spring **122** may act against a portion of bearing surface **206y** that is radially inset from the portion of bearing surface **206z** that contacts first spring **220** (*see* FIG. 28). Stated another way, first spring **220** may act against a radially outset surface and second spring **222** may act against a radially inset surface, at least with respect to one another. Each of springs **220, 222** may have a relative stiffness of about 1 N/mm - 6 N/mm, more particularly about 2 N/mm - 4N/mm, and even more particularly about 3.35 N/mm. In some embodiments, only the second spring **222** contributes a significant portion of the relative stiffness ranges provided above and the first spring **220** may have a negligible contribution. In other embodiments, only the first spring **220** contributes a significant portion of the relative stiffness ranges provided above and the second spring **222** may have a negligible contribution. In other embodiments still, the sum of the stiffness of the first and second springs **220, 222** may be additive such that the combined stiffness of the first and second springs **220, 222** may be within the stiffness ranges provided above. At least one advantage of the first and/or second springs **220, 222** is that they may facilitate the clipping of a bone screw **300** to retaining cap **208** and the extraction of the bone screw **300** from the retaining cap **208** in a progressive manner. For example, when driving a bone screw **300** into a target site, the first and/or second springs **220, 222** may compress when the bone screw **300** nears a fully anchored position helping to moderate the extraction force required to remove the bone screw **300** in a precise or controlled way, or at least a relatively more precise or controlled way relative to conventional screw drivers. For example still, the first and/or second springs **220, 222** may allow the retaining cap **208** to progressively release a bone screw **300** when it is inserted and installed into a cavity or target location in a similar way.

FIG. 29 illustrates an alternate embodiment showing the retaining cap **208** including a retaining ring **208r.** Retaining ring **208r** may be formed of the same or similar materials as previously disclosed with respect to retaining cap **208.** In at least one embodiment, retaining ring **208r** is formed of a metallic material and retaining cap **208** is formed of PEEK. Retaining ring **208r** may have a generally toroidal shape or torus shape. The retaining ring **208r** may have at least one expansion joint **208e** that divides the retaining ring into separable spaced apart regions. The expansion joint **208e** may allow the retaining ring **208r** to expand radially when a bone screw **300** is insert into the retaining cap **208.** For example, the retaining ring **208r** may have an internal radius and an external radius and the internal radius may correspond to the radius of a head of a bone screw **300.** In the disclosed embodiment, the internal radius of the retaining ring **208r** may be slightly less than the radius of a head of a bone screw **300** and may expand radially when a bone screw **300** is insert therein. In the disclosed embodiment, bone screw **300** includes an indent **310** (*see* FIGS. 19-22). In a head on view, the indent **310** may be defined by a radius of a circle that is slightly less than a radius defining a tip portion of the head of bone screw **300.** The retaining ring **208r** may have an internal radius that corresponds to the radius of the indent **310.** Additionally, when viewed in a cross sectional view, retaining ring **208r** may have a diameter that corresponds to a depth of indent **310** and the retaining ring **208r** may be half-seated within the indent **310.** For example, when the retaining ring **208r** is seated in the indent **310,** about half of the retaining ring **208r** extends above the indent **310** and the other half of retaining ring **208r** is within indent **310.** However, in other embodiments the retaining ring **208r** may be fully seated or partially seated in indent **310,** *e.g.,* 100% seated, 75% seated, or 25% seated.

FIG. 16 illustrates an alternate embodiment where retaining cap **208** includes a protrusion **208x** (or a lip portion). Protrusion **208x** may be a rounded or arcuate protrusion that extends circumferentially around the inside of retaining cap **208.** For example, when viewed in cross section, protrusion **208x** may be defined by a radius that is relatively smaller than a radius of the head of bone screw **300.** At least one advantage of protrusion **208x** is that it may assist in retaining bone screw **300** inside of retaining cap **208.** Furthermore, in some embodiments, protrusion **208x** may serve a similar function and/or have similar functionality as bump **208c.** At least one advantage to protrusion **208x** is that conventional bone screws that do not include indent **310** may be safely retained and controllably released by retaining cap **208** similarly as explained above with respect to bump **208c.** In some embodiments, retaining cap **208** may combine both bump **208c** and protrusion **208x.**

FIG. 31 illustrates an alternate embodiment including a joint mechanism **205** in lieu of gear mechanism **203.** Joint mechanism **205** may be operable/drivable via drive shaft **202** in the same, similar, or substantially the same way as gear mechanism **203** as explained above. As illustrated in FIG. 31, joint mechanism **205** may include a spherical portion **205a** that is rotatably seated in a spherical housing area of tip portion **206.** Spherical portion **205a** may be fixedly coupled to drive portion **205b** and drive portion **205b** may include an end portion configured for driving bone screw **300** in the same, similar, or substantially the same way as drive end **202b** explained above. In the example embodiment, spherical portion **205a** includes an aperture **205c** such as a drilled out portion or slotted portion configured to receive drive shaft **202** therein, for example. Drive shaft **202** may extend into aperture **205c** and couple with spherical portion **205a** and/or drive portion **205b** to transmit rotational movement from drive shaft **202** to bone screw **300.** For example drive shaft **202** may extend into aperture **205c** and couple with spherical portion **205a** and/or drive portion **205b** via a pin connection. At least one advantage of this structural arrangement is that the joint mechanism **205** may be configured such that it is adjustable to accommodate a range of various angles of inclination β of tip portion **206** with respect to housing **110.** For example, joint mechanism **205** may enable a user selectable and greater operating range of various angles of inclination β. For example still, joint mechanism **205** may enable tip portion **206** to be adjustably inclined within a range of about 20° - 60°, and more particularly about 30° - 50 with respect to a longitudinal direction of housing **110.** However, it shall be understood that in some embodiments the angle of inclination β may be fixed.

FIG. 31 also illustrates an alternate embodiment of retaining cap **208.** In the disclosed embodiment, retaining cap **208** may include a tapered portion **208t.** For example, the bone screw connecting side **208bs** of retaining cap **208** may taper conically. For example still, when viewed in cross-section, a radius of tapered portion **208t** may progressively decrease along retaining cap **208** to an outermost end thereof. It shall be understood that retaining cap **208** may include bump **208c,** protrusion **208x,** and/or tapered portion **208t** and any combination thereof. Additionally, any of bump **208c,** protrusion **208x,** and/or tapered portion **208t** may be referred to as a retaining contour or retaining feature in some embodiments.

Example screwdriver **200** may be operably coupled to a manual hand driver **401** and/or powered driver **400** as explained above with respect to drill **100.** Hand driver **401** may selectively couple and uncouple with drive portion **202a** of drive shaft **202,** for example. Hand driver may be of a fixed type or a ratcheting type. At least one example of a manual hand driver **401** may be the commercially available Medtronic QC handle. Example screwdriver **200** may also be operably coupled to a powered driver **400** in accordance with the principles of the present disclosure. Powered driver **400** may be powered by any means, *e.g*., electrically operated or pneumatically operated as explained above with respect to drill **100.**

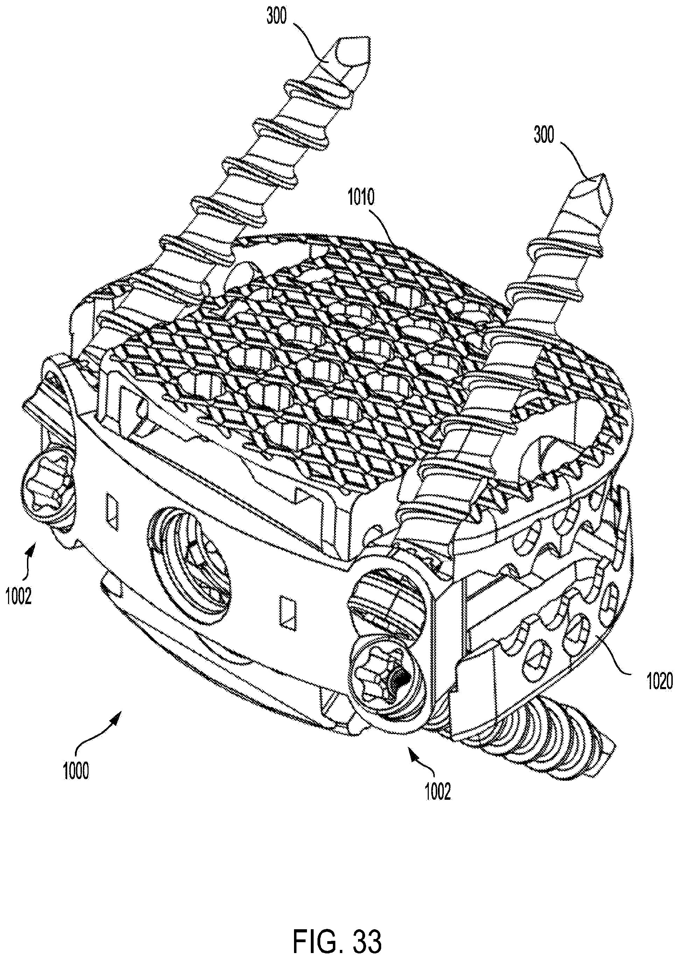

FIG. 33 is a perspective view of an example medical device **1000** that includes a plurality of inclined bone screw apertures **1001** . Consistent with the principles of the disclosure, various example screwdrivers **200** may include a tip portion **206** that is angled at a degree β with respect to a longitudinal direction of housing **110.** In some embodiments, tip portion **206** may be angled such that the degree β corresponds to the desired inclination of bone screw **300** and/or bone screw apertures **1001 .** Additionally, tip portion **206** may be angled at a degree β that accounts for both (1) the desired inclination of bone screw **300** and/or bone screw apertures **1001** ; and (2) the particular type of surgery (anterior, lateral, oblique, etc.) and surgical access opening available to a surgeon.

## Claims

1. A screwdriver (200, 400), comprising:
a rotatable drive shaft (202), the drive shaft (202) including a drive portion (202a) disposed at a distal end thereof, a drive end (202b) disposed at a proximal end thereof, and a main shaft portion (202c) extending in a longitudinal direction through a housing (110);
a movable handle mechanism (150) coupled to and disposed at a medial portion of the housing (110);
an angled tip portion (206) disposed at the proximal end, the angled tip portion being angled with respect to the longitudinal direction, the drive end (202b) of the drive shaft (202) extending through the angled tip portion (206);
a mechanism (203) configured to transfer a rotational force applied to the drive portion (202a) of the drive shaft (202) through the angled tip portion (206) to the drive end (203) of the drive shaft (202);
an elastic retaining clip (208) configured to have a bone screw (300) securely attached therein at a clipping force and progressively release the bone screw (300) therein at an extraction force, the elastic retaining clip (208) being removably and operably coupled with the drive end (202b) of the drive shaft (202); and
a first spring (222) contacting the elastic retaining clip (208) and the angled tip portion (206), the first spring (222) being configured to facilitate the progressive release of the bone screw (300).

2. The screwdriver (200, 400) of claim 1, wherein the movable handle mechanism (150) includes a positioning handle (104).

3. The screwdriver (200, 400) of claim 1, wherein the movable handle mechanism (150) is configured to move forward and backward in a longitudinal direction along the housing (110).

4. The screwdriver (200, 400) of claim 3, wherein the housing (110) includes a plurality of channels (111) and the movable handle mechanism (150) is configured to securely couple to the housing (110) via at least one channel of the plurality of channels (111).

5. The screwdriver (200, 400) of claim 1, wherein the movable handle mechanism is configured to rotate clockwise and counterclockwise around the housing (110).

6. The screwdriver (200, 400) of claim 5, wherein:
the housing (110) includes a plurality of detents (112), and
the movable handle mechanism (150) is configured to securely couple to the housing (110) via at least one detent of the plurality of detents (112).

7. The screwdriver (200, 400) of claim 6, wherein: the movable handle mechanism (150) further comprises a positioning ball (154) having a size and shape corresponding to a size and shape of a detent of the plurality of detents (112), the positioning ball (154) being configured to securely couple the movable handle mechanism (150) via one detent of the plurality of detents (112).

8. The screwdriver (200, 400) of claim 1, wherein:
the movable handle mechanism (150) is configured to move forward and backward in a longitudinal direction along the housing (110) between a proximal stop ring (113) and a distal stop ring (114),
the movable handle mechanism (150) is configured to rotate clockwise and counterclockwise around the housing (110),
the housing includes a plurality of channels (111), each channel of the plurality of channels (111) including at least one detent (112), and
the movable handle mechanism (150) is configured to securely couple to the housing (110) via at least one channel of the plurality of channels (111) and at least one detent of the plurality of detents (112).

9. The screwdriver (200, 400) of claim 1, further comprising a second spring (220) configured to facilitate the progressive release of the bone screw (300),
wherein the first spring (222) and second spring (220) each contact the elastic retaining clip (208) and the angled tip portion (206), and
wherein the first spring (222) is disposed, at least partly, within a central cavity of the second spring (220).

10. The screwdriver (200, 400) of claim **1,** wherein:
the elastic retaining clip (208) further comprises at least one protrusion (208x) disposed at an end portion thereof configured to facilitate the retention of the bone screw (300), and
the at least one protrusion (208x) is radially inset with respect to a head portion of the bone screw and is configured to contact an end portion of the head portion of the bone screw (300) to thereby facilitate the retention of the bone screw (300).

## Patentansprüche

1. Schraubendreher (200, 400), umfassend:
eine drehbare Antriebswelle (202), wobei die Antriebswelle (202) einen Antriebsabschnitt (202a), der an einem distalen Ende davon angeordnet ist, ein Antriebsende (202b), das an einem proximalen Ende davon angeordnet ist, und einen Hauptwellenabschnitt (202c) einschließt, der sich in einer Längsrichtung durch ein Gehäuse (110) hindurch erstreckt;
einen bewegbaren Griffmechanismus (150), der mit einem mittleren Abschnitt des Gehäuses (110) gekoppelt und an diesem angeordnet ist;
einen abgewinkelten Spitzenabschnitt (206), der an dem proximalen Ende angeordnet ist, wobei der abgewinkelte Spitzenabschnitt in Bezug auf die Längsrichtung abgewinkelt ist, wobei sich das Antriebsende (202b) der Antriebswelle (202) durch den abgewinkelten Spitzenabschnitt (206) hindurch erstreckt;
einen Mechanismus (203), der konfiguriert ist, um eine Drehkraft, die auf den Antriebsabschnitt (202a) der Antriebswelle (202) ausgeübt wird, durch den abgewinkelten Spitzenabschnitt (206) auf das Antriebsende (203) der Antriebswelle (202) zu übertragen;
eine elastische Halteklammer (208), die konfiguriert ist, um eine Knochenschraube (300) mit einer Klemmkraft sicher darin befestigt aufzuweisen und die Knochenschraube (300) darin bei einer Ausziehkraft progressiv freizugeben, wobei die elastische Halteklammer (208) lösbar und mit dem Antriebsende (202b) der Antriebswelle (202) wirkgekoppelt ist; und
eine erste Feder (222), die den elastischen Halteclip (208) und den abgewinkelten Spitzenabschnitt (206) kontaktiert, wobei die erste Feder (222) konfiguriert ist, um die schrittweise Freigabe der Knochenschraube (300) zu erleichtern.

2. Schraubendreher (200, 400) nach Anspruch 1, wobei der bewegbare Griffmechanismus (150) einen Positioniergriff (104) einschließt.

3. Schraubendreher (200, 400) nach Anspruch 1, wobei der bewegbare Griffmechanismus (150) konfiguriert ist, um sich in einer Längsrichtung entlang des Gehäuses (110) vorwärts und rückwärts zu bewegen.

4. Schraubendreher (200, 400) nach Anspruch 3, wobei das Gehäuse (110) eine Vielzahl von Kanälen (111) einschließt und der bewegbare Griffmechanismus (150) konfiguriert ist, um über mindestens einen Kanal der Vielzahl von Kanälen (111) mit dem Gehäuse (110) sicher gekoppelt zu werden.

5. Schraubendreher (200, 400) nach Anspruch 1, wobei der bewegbare Griffmechanismus konfiguriert ist, um sich im Uhrzeigersinn und gegen den Uhrzeigersinn um das Gehäuse (110) herum zu drehen.

6. Schraubendreher (200, 400) nach Anspruch 5, wobei:
das Gehäuse (110) eine Vielzahl von Arretierungen (112) einschließt, und
der bewegbare Griffmechanismus (150) konfiguriert ist, um über mindestens eine Arretierung der Vielzahl von Arretierungen (112) mit dem Gehäuse (110) sicher zu koppeln.

7. Schraubendreher (200, 400) nach Anspruch 6, wobei: der bewegbare Griffmechanismus (150) ferner eine Positionierkugel (154) umfasst, die eine Größe und Form aufweist, die einer Größe und Form einer Arretierung der Vielzahl von Arretierungen (112) entsprechen, wobei die Positionierkugel (154) konfiguriert ist, um den bewegbaren Griffmechanismus (150) über eine Arretierung der Vielzahl von Arretierungen (112) sicher zu koppeln.

8. Schraubendreher (200, 400) nach Anspruch 1, wobei:
der bewegbare Griffmechanismus (150) konfiguriert ist, um sich in einer Längsrichtung entlang des Gehäuses (110) zwischen einem proximalen Anschlagring (113) und einem distalen Anschlagring (114) vorwärts und rückwärts zu bewegen,
der bewegbare Griffmechanismus (150) konfiguriert ist, um sich im Uhrzeigersinn und gegen den Uhrzeigersinn um das Gehäuse (110) herum zu drehen,
das Gehäuse eine Vielzahl von Kanälen (111) einschließt, wobei jeder Kanal der Vielzahl von Kanälen (111) mindestens eine Arretierung (112) einschließt, und
der bewegbare Griffmechanismus (150) konfiguriert ist, um über mindestens einen Kanal der Vielzahl von Kanälen (111) und mindestens eine Arretierung der Vielzahl von Arretierungen (112) mit dem Gehäuse (110) sicher zu koppeln.

9. Schraubendreher (200, 400) nach Anspruch 1, ferner umfassend eine zweite Feder (220), die konfiguriert ist, um die schrittweise Freigabe der Knochenschraube (300) zu erleichtern,
wobei die erste Feder (222) und die zweite Feder (220) jeweils den elastischen Halteclip (208) und den abgewinkelten Spitzenabschnitt (206) zu kontaktieren, und
wobei die erste Feder (222), mindestens teilweise, innerhalb eines zentralen Hohlraums der zweiten Feder (220) angeordnet ist.

10. Schraubendreher (200, 400) nach Anspruch 1, wobei:
der elastische Halteclip (208) ferner mindestens einen Vorsprung (208x) umfasst, der an einem Endabschnitt davon angeordnet und konfiguriert ist, um den Halt der Knochenschraube (300) zu erleichtern, und
der mindestens eine Vorsprung (208x) in Bezug auf einen Kopfabschnitt der Knochenschraube radial nach innen versetzt ist und konfiguriert ist, um einen Endabschnitt des Kopfabschnitts der Knochenschraube (300) zu kontaktieren, um dadurch das Zurückhalten der Knochenschraube (300) zu erleichtern.

## Revendications

1. Tournevis (200, 400) comprenant :
un arbre d'entraînement rotatif (202), l'arbre d'entraînement (202) comportant une partie d'entraînement (202a) disposée à une extrémité distale de celui-ci, une extrémité d'entraînement (202b) disposée à une extrémité proximale de celui-ci, et une partie d'arbre principal (202c) s'étendant dans une direction longitudinale à travers un boîtier (110) ;
un mécanisme de poignée mobile (150) accouplé au boîtier (110) et disposé au niveau d'une partie médiane de celui-ci ;
une partie de pointe inclinée (206) disposée à l'extrémité proximale, la partie de pointe inclinée étant inclinée par rapport à la direction longitudinale, l'extrémité d'entraînement (202b) de l'arbre d'entraînement (202) s'étendant à travers la partie de pointe inclinée (206) ;
un mécanisme (203) conçu pour transférer une force de rotation appliquée à la partie d'entraînement (202a) de l'arbre d'entraînement (202) à travers la partie de pointe inclinée (206) à l'extrémité d'entraînement (203) de l'arbre d'entraînement (202) ;
une pince de retenue élastique (208) conçue pour qu'une vis à os (300) y soit solidement fixée à une force de pincement et pour libérer progressivement la vis à os (300) s'y trouvant à une force d'extraction, la pince de retenue élastique (208) étant accouplée de manière amovible et fonctionnelle à l'extrémité d'entraînement (202b) de l'arbre d'entraînement (202) ; et
un premier ressort (222) en contact avec la pince de retenue élastique (208) et la partie de pointe inclinée (206), le premier ressort (222) étant conçu pour faciliter la libération progressive de la vis à os (300).

2. Tournevis (200, 400) selon la revendication 1, dans lequel le mécanisme de poignée mobile (150) comporte une poignée de positionnement (104).

3. Tournevis (200, 400) selon la revendication 1, dans lequel le mécanisme de poignée mobile (150) est conçu pour se déplacer vers l'avant et vers l'arrière dans une direction longitudinale le long du boîtier (110).

4. Tournevis (200, 400) selon la revendication 3, dans lequel le boîtier (110) comporte une pluralité de canaux (111) et le mécanisme de poignée mobile (150) est conçu pour s'accoupler solidement au boîtier (110) par l'intermédiaire d'au moins un canal de la pluralité de canaux (111).

5. Tournevis (200, 400) selon la revendication 1, dans lequel le mécanisme de poignée mobile est conçu pour tourner dans le sens horaire et dans le sens antihoraire autour du boîtier (110).

6. Tournevis (200, 400) selon la revendication 5, dans lequel :
le boîtier (110) comporte une pluralité de crans (112), et
le mécanisme de poignée mobile (150) est conçu pour s'accoupler solidement au boîtier (110) par l'intermédiaire d'au moins un cran de la pluralité de crans (112).

7. Tournevis (200, 400) selon la revendication 6, dans lequel : le mécanisme de poignée mobile (150) comprend en outre une bille de positionnement (154) présentant une taille et une forme correspondant à une taille et une forme d'un cran de la pluralité de crans (112), la bille de positionnement (154) étant conçue pour accoupler solidement le mécanisme de poignée mobile (150) par l'intermédiaire d'un cran de la pluralité de crans (112).

8. Tournevis (200, 400) selon la revendication 1, dans lequel :
le mécanisme de poignée mobile (150) est conçu pour se déplacer vers l'avant et vers l'arrière dans une direction longitudinale le long du boîtier (110) entre une bague de butée proximale (113) et une bague de butée distale (114),
le mécanisme de poignée mobile (150) est conçu pour tourner dans le sens horaire et dans le sens antihoraire autour du boîtier (110),
le boîtier comporte une pluralité de canaux (111), chaque canal de la pluralité de canaux (111) comportant au moins un cran (112), et
le mécanisme de poignée mobile (150) est conçu pour s'accoupler solidement au boîtier (110) par l'intermédiaire d'au moins un canal de la pluralité de canaux (111) et d'au moins un cran de la pluralité de crans (112).

9. Tournevis (200, 400) selon la revendication 1, comprenant en outre un second ressort (220) conçu pour faciliter la libération progressive de la vis à os (300),
dans lequel le premier ressort (222) et le second ressort (220) sont chacun en contact avec l'attache de retenue élastique (208) et la partie de pointe inclinée (206), et
dans lequel le premier ressort (222) est disposé, au moins partiellement, dans une cavité centrale du second ressort (220).

10. Tournevis (200, 400) selon la revendication 1, dans lequel :
la pince de retenue élastique (208) comprend en outre au moins une saillie (208x) disposée au niveau d'une partie d'extrémité de celui-ci conçue pour faciliter la retenue de la vis à os (300), et
l'au moins une saillie (208x) est en retrait radialement par rapport à une partie de tête de la vis à os et est conçue pour entrer en contact avec une partie d'extrémité de la partie de tête de la vis à os (300) pour faciliter ainsi la retenue de la vis à os (300).
